# EUROPÄISCHE PATENTSCHRIFT

(11) **EP 1 280 771 B1**
(45) Veröffentlichungstag und Bekanntmachung des Hinweises auf die Patenterteilung: **13.10.2004**
(21) Anmeldenummer: 01940388.0
(22) Anmeldetag: 02.05.2001
(51) Int. Cl.: C07D 209/04, C07D 209/60, C07H 15/26, A61K 31/70, A61K 31/40, A61K 31/47, C07H 15/24, C07H 15/203, C07D 487/04

(54) **NEUE PRODRUGS VON 6-HYDROXY-2,3-DIHYDRO-1H-INDOLEN, 5-HYDROXY-1,2-DIHYDRO-3H-PYRROLO 3,2-E]INDOLEN UND 5-HYDROXY-1,2-DIHYDRO-3H-BENZO E]INDOLEN SOWIE VON 6-HYDROXY-1,2,3,4-TETRAHYDRO-BENZO F]CHINOLIN-DERIVATEN FÜR EINE SELEKTIVE KREBSTHERAPIE**
NOVEL PRODRUGS VON 6-HYDROXY-2,3-DIHYDRO-1H-INDOLES, 5-HYDROXY-1,2-DIHYDRO-3H-PYRROLO 3,2-E]INDOLES AND 5-HYDROXY-1,2-DIHYDRO-3H-BENZO(E)INDOLES AS WELL AS OF 6-HYDROXY-1,2,3,4-TETRAHYDRO-BENZO F]QUINOLINE DERIVATIVES FOR USE IN SELECTIVE CANCER THERAPY
NOUVEAUX PROMEDICAMENTS DE 6-HYDROXY-2,3-DIHYDRO-1H-INDOLES, DE 5-HYDROXY-1,2-DIHYDRO-3H-PYRROLO 3,2-E]INDOLES ET DE 5-HYDROXY-1,2-DIHYDRO-3H-BENZO(E)INDOLES AINSI QUE DE DERIVES DE 6-HYDROXY-1,2,3,4-TETRAHYDRO-BENZO F]QUINOLINE DESTINES A UNE THERAPIE ANTICANCEREUSE SELECTIVE

(30) Priorität: 02.05.2000 DE 10021335; 23.05.2000 DE 10025329
(43) Veröffentlichungstag der Anmeldung: 05.02.2003
(73) Patentinhaber: Tietze, Lutz F., Prof. Dr., 37077 Göttingen (DE)
(72) Erfinder: Tietze, Lutz, F., Inst. für Organische Chemie, 37077 Göttingen (DE); Fecher, Anja, Kingston, Ontario K7K 1M8 (CA); Herzig, Tobias, 37075 Göttingen (DE)
(74) Vertreter: Eisenführ, Speiser & Partner
(86) Internationale Anmeldenummer: PCT/EP2001/004904
(87) Internationale Veröffentlichungsnummer: WO 2001/083448

(56) Entgegenhaltungen:
- WO-A-97/44000
- WO-A-98/11101
- US-A- 5 646 298
- TIETZE, L.F. ET AL.: "Prodrugs of the cytostatic CC-1065 that can be activated in a tumor-selective manner" ANGEW. CHEM. INT. ED., Bd. 35, 1996, Seiten 2674-7, XP002183014 in der Anmeldung erwähnt
- LING, LEI ET AL: "A practical route to optically active CBI, a potent analog of the CC-1065 alkylation subunit" HETEROCYCL. COMMUN. (1997), 3(5), 405-408 , 1997, XP001055518
- MURATAKE, HIDEAKI ET AL: "A novel phenol-forming reaction for preparation of benzene, furan, and thiophene analogs of CC-1065/duocarmycin pharmacophores" TETRAHEDRON LETT. (1997), 38(43), 7577-7580 , 1997, XP004125300
- ARISTOFF, PAUL A. ET AL: "Synthesis and biochemical evaluation of the CBI-PDE-I-dimer, a benzannelated analog of (+)-CC-1065 that also produces delayed toxicity i mice" J. MED. CHEM. (1993), 36(14), 1956-63 , 1993, XP000910185

## Beschreibung

Es ist bekannt, daß die zur Zeit verfügbaren Krebschemotherapeutika aufgrund ihrer geringen Selektivität unerwünschte Nebenwirkungen verursachen, die dosislimitierend sind, oder zu einem Abbruch der Therapie führen können. Das Ziel muß es daher sein, genetische und phenotypische Unterschiede zwischen normalen und malignen Zellen zu nutzen, um eine höhere Selektivität zu erzielen.

Hierbei können Prodrugs eingesetzt werden, die entweder säurekatalysiert durch den in Tumorzellen um 1.2 Einheiten erniedrigten pH-Wert oder im Rahmen des ADEPT-Konzeptes (*antibody direcred enzyme prodrug therapy*) gespalten werden, das auf der Nutzung von Konjugaten aus Glykohydrolasen und monoklonalen Antikörpern basiert, die an tumorassoziierte Antigene binden [vgl. Pharmacology & Therapeutics 83, 67-123, (1999); J. Biol. Chem. 272, 15804-15816, (1997); J. Med. Chem. 41, 1507-1512, (1998); Bioconjug. Chem. 98, 255-259, (1998); Cancer Immunol. Immunother. 44, 3 05-315, (1997)]. Dabei werden zytotoxische Verbindungen eingesetzt, die durch Überführung in enzymatisch spaltbare Derivate (Prodrugs) detoxifiziert werden. Die Enzym-Antikörper-Konjugate liegen nach der Verabreichung selektiv an der Oberfläche der Krebszellen vor und bewirken nur im Tumor eine Spaltung der Prodrugs unter Freisetzung der zytotoxischen Verbindung.

Als zytotoxische Verbindungen wurden u.a. bereits Derivate des *seco*-CI-Analogons des Naturstoffs CC-1065 verwendet und in entsprechende Prodrugs u.a. als Galaktoside umgewandelt. Für die Antikörper-Enzym-Konjugate wurden u.a. Glycohydrolasen benutzt [vgl. Angew. Chem. 108, 2840-2842, (1996); Angew. Chem. Int. Ed. Engl. 35, 2674-2677, (1996)]. Wesentliche Kriterien für die erfolgreiche Verwendung von Prodrugs im Rahmen des ADEPT-Konzeptes sind die geringe Toxizität der Prodrugs, eine sehr hohe Zytotoxizität des zugrunde liegenden Zytostatikums und eine schnelle Spaltung des Prodrugs in Gegenwart des entsprechenden Enzyms.

Es ist weiterhin bekannt, daß maligne Zellen eine gegenüber dem Normalgewebe erhöhte Glycolyse und damit Lactatproduktion aufzeigen und der pH-Wert im Tumorgewebe durch intravenös verabreichte Glucose gesenkt werden kann [vgl. S. Tanneberger, Experimentelle und klinische Tumorchemotherapie; Allgemeine Tumorchemotherapie; G. Fischer Verlag, Stuttgart/New York 1980; Naturwiss. 46, 2(1959); Cancer Res. 42, 1498 (1982); 42, 1505 (1982); 49, 4373 (1989)].

Es ist auch bekannt, daß zahlreiche Glykohydrolasen im leicht sauren Milieu eine höhere Aktivität, als bei pH 7.4 zeigen. Diese können außerdem an monoklonale Antikörper gebunden werden, welche selektiv an spezifische tumorassoziierte Antigene auf der Membran von malignen Zellen binden [vgl. Pharmacology & Therapeutics 83, 67 (1999)].

In der Vergangenheit wurde versucht, diese Unterschiede im pH-Wert zwischen normalem und Tumorgewebe für eine selektive Tumortherapie auszunutzen [vgl. Cancer Res. *49*, 4179, (1989); Liebigs Ann. Chem. 847 (1987); Tetrahedron Lett. *22*, 239, (1981); Angew. Chem. *102*, 812, (1990); Liebigs Ann. Chem. 151, (1990)]. Die hierbei zum Einsatz gekommenen säurelabilen untoxischen Prodrugs von alkylierenden Verbindungen erwiesen sich jedoch als nicht ausreichend säurelabil, als daß sie selektiv im Tumorgewebe zum aktiven cytociden Agens gespalten wurden.

Es wurde ebenfalls versucht, zytotoxische Verbindungen durch Derivatisierung in enzymatisch spaltbare Prodrugs mit verminderter Toxizität zu überfübren. Bei den bisher hergestellten Verbindungen bestand das Problem, daß entweder die Unterschiede der Zytotoxizität von Prodrug und dem zugrundeliegenden Drug nicht ausreichend war, oder/und das Drug keine ausreichende Wirksamkeit aufwies [vgl. Angew. Chem. *108*, 2840, (1996)]. Im Gegensatz hierzu zeigen die in dieser Erfindung vorgestellten säure-katalysiert oder vorzugsweise mit Glycohydrolasen enzymatisch spaltbaren Prodrugs von 6-Hydroxy-2,3-dihydro-1H-indolen, 5-Hydroxy-1,2-dihydro-3H-pyrrolo[3,2-e]indolen und 5-Hydroxy-1,2-dihydro-3H-benzo[e]indolen sowie von 6-Hydroxy-1,2,3,4-tetrahydro-benzo[*f*]-chinolin-Derivaten unerwarteterweise eine bisher nicht gefundene Selektivität von über 1: 1500 zwischen Prodrug und dem dem Prodrug zugrundeliegenden Zytostatikum bei gleichzeitiger hoher Zytotoxizität des Zytostatikums in der Zellkultur.

Die vorliegende Erfindung betrifft neuartig substituierte 6-Hydmxy-2,3-dihydro-1H-indole der allgemeinen Formel (I), neuartig substituierte 5-Hydroxy-1,2-dihydro-3Hpyrrolo[3,2-e]indole der allgemeinen Formel (II) und neuartig substituierte 5-Hydroxy-1,2-dihydro-3H-benzo[e]indole der allgemeinen Formel (III) sowie deren O-Glykoside mit Mono-, Di- und Oligosacchariden der allgemeinen Formeln (IV), (V) und (VI) und neuartige O-Glykoside mit Mono-, Di- und Oligosacchariden von 6-Hydroxy-1,2,3,4-tetrahydro-benzo[*f*]-chinoline der allgemeinen Formeln (VII) sowie 6-Hydroxy-1,2,3,4-tetrahydro-benzo[*f*]-chinoline der allgemeinen Formel (VIII) nach Anspruch 1, Verfahren zu ihrer Herstellung nach den Ansprüchen 4 und 8 und ihre Verwendung als hochselektive Zytostatika in der Krebstherapie nach den Ansprüchen 9 und 10.

Vorteilhafte Ausführungsformen der neuen Verbindungen und des neuen Verfahrens sind in den Unteransprüchen 2 und 3 bzw. 5 bis 7 beschrieben.

Hydroxyschutzgruppe steht im Rahmen der Erfindung allgemeinen für eine Schutzgruppe aus der Reihe: *tert*.-Butoxydiphenylsilyl, Trimethylsilyl, Triethylsilyl, Triisopropylsilyl, *tert*.-Butyldimethylsilyl, *tert*.-Butyldiphenylsilyl, Triphenylsilyl, Tri- methylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, *tert*.-Butyloxycarbonyl, Allyl- oxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methylthiomethyl, Methoxyethoxymethyl, [2-(Trimethylsilyl)ethoxy]methyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Benzoyl, Benzyl oder Methylbenzyl. Aminoschutzgruppen im Rahmen der Erfindung sind die üblichen in der Peptid-Chemie verwendeten Aminoschutzgruppen. Hierzu gehören bevorzugt: Benzyloxycarbonyl, 3,4-Dimethoxybenzyloxycarbonyl, 3,5-Dimethoxybenzyloxycarbanyl, 2,4-Dimethoxybenzyloxycarbonyl, 4-Methoxybenzyloxy- carbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Iso propoxycarbonyl, Butoxycarbonyl, Isobutoxycarbonyl, *tert*.-Butaxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlortertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl. Bevorzugt sind Benzyloxycarbonyl, *tert*.-Butoxycarbonyl und Acetyl.

Das erfindungsgemäße Verfahren zur Herstellung der neuen Verbindungen der allgemeinen Formeln (I), (II), (III), (IV), (V) und (VI) nach Anspruch 4 kann durch folgende Formelschemata beispielhaft erläutert werden:

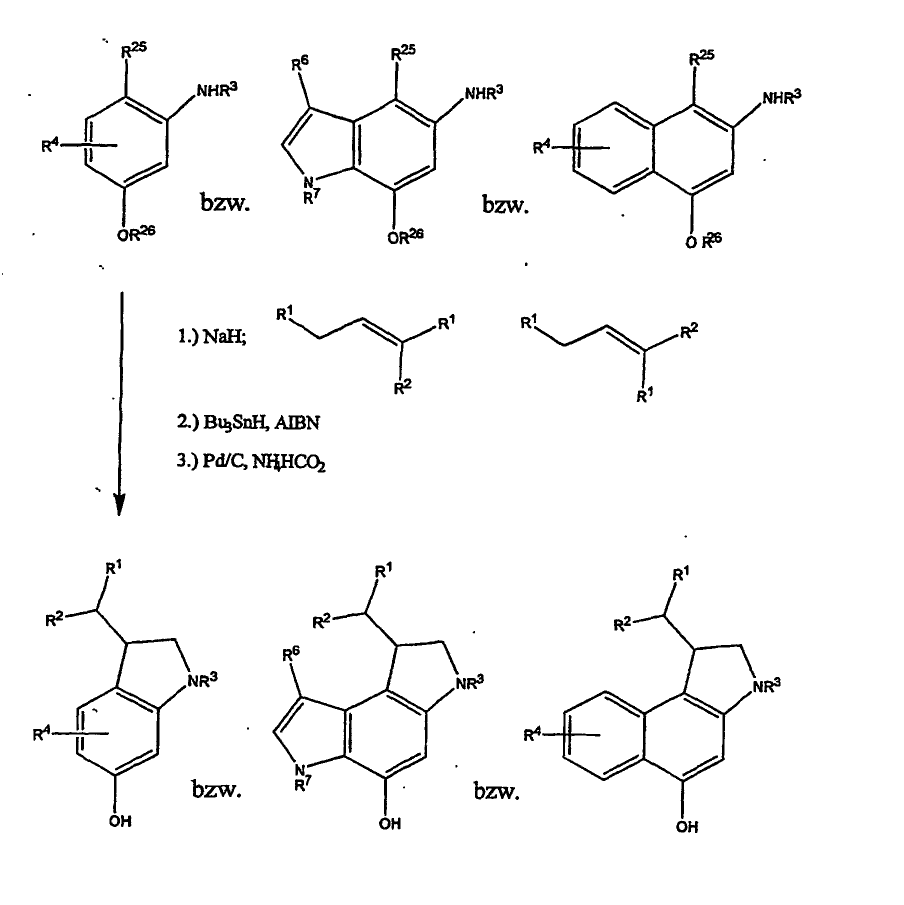

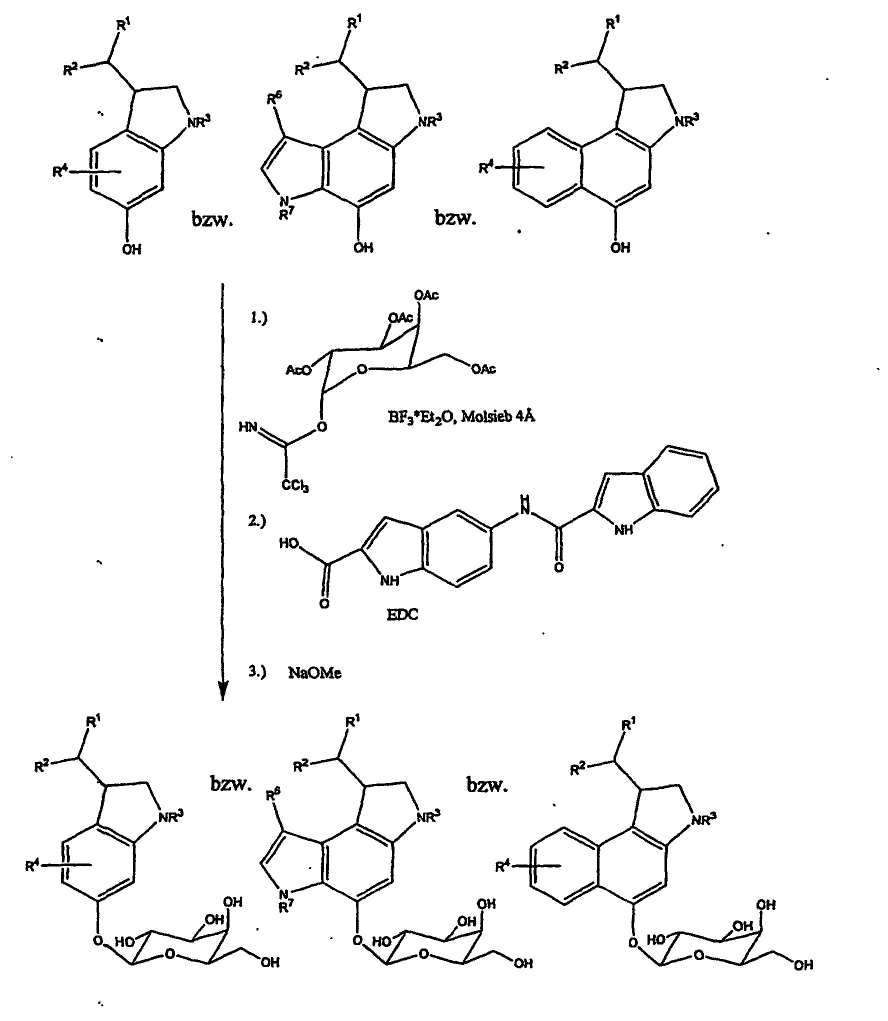

Das erfindungsgemäße Verfahren zur Herstellung der neuen Verbindungen der allgemeinen Formel (VII) kann durch folgendes Formelschema beispielhaft erläutert werden:

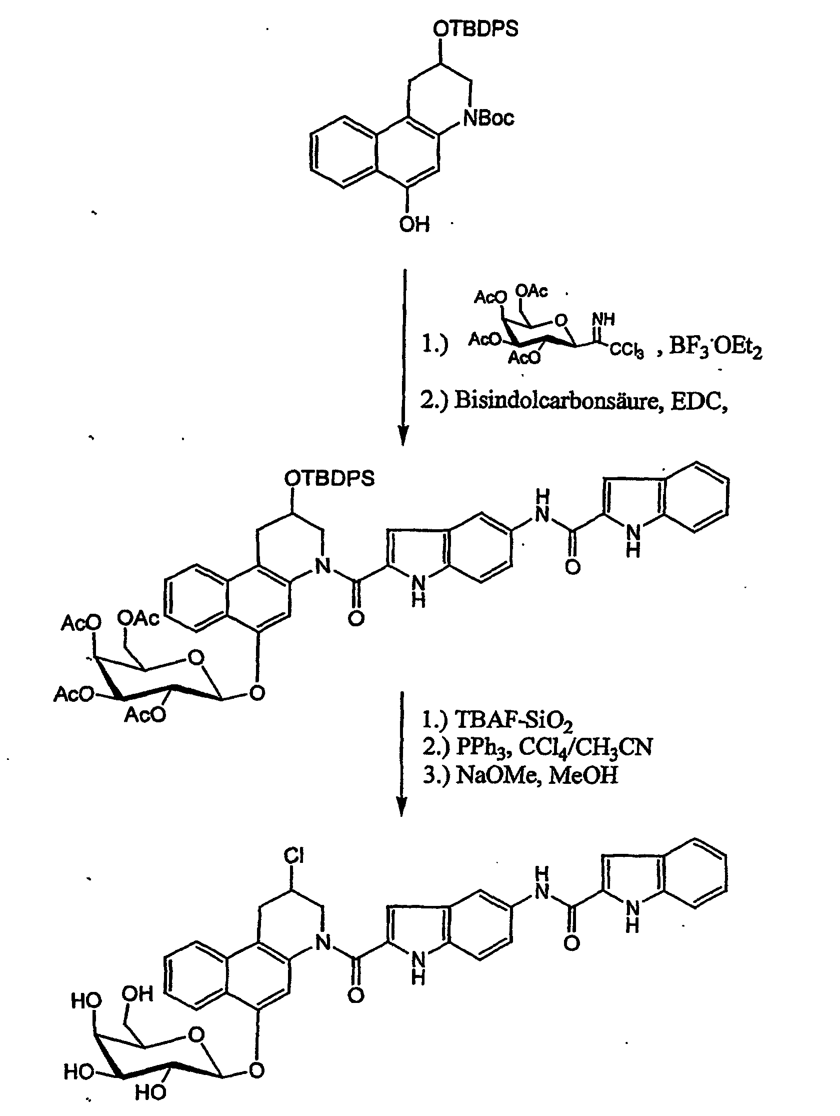

Geeignete Basen zur Deprotonierung im oben angegebenen Sinne sind Alkali- und Erdalkalimetallhydride, vorzugsweise Natriumhydrid. Als Lösemittel sind aprotische organische Lösemittel geeignet, beispielsweise Dimethylformamid, Tetrahydrofuran oder Diethylether. Bevorzugt ist Dimethylformamid. Die Reaktion verläuft in einem Temperaturbereich von -30°C bis 80°C unter Normaldruck, vorzugsweise bei 0°C bis 80°C Die Cyclisierung erfolgt bei Normaldruck unter Argonatmosphäre in einem Temperaturbereich von 50°C bis 110°C, vorzugsweise bei 80°C bis 110°C, in Gegenwart von Tributylzinnhydrid. Geeignete Lösemittel sind aromatische Kohlenwasserstoffe, bevorzugt Benzol und Toluol. Als Radikalinitiator ist α,α'-Azo-isobutyronitril bevorzugt.

Die Abspaltung der Schutzgruppen am phenolischen Sauerstoff erfolgt durch hydrogenolytische Spaltung, im Fall der Benzylgruppe in Anwesenheit eines Katalysators, beispielsweise ein Gemisch aus Palladium/C und Palladium/CaCO₃, mit Wasserstoff oder durch Zusatz von Ammoniumformiat, in einem der oben aufgeführten Lösemittel, vorzugsweise Tetrahydrofuran, Methanol oder Aceton, in einem Temperaturbereich von 0°C bis 60°C, vorzugsweise bei 20°C bis 50°C oder durch Lithium oder Natrium in flüssigem Ammoniak.

Zur Herstellung der glycosidierten Prodrugs wird das freie Phenol mit einem Glycosyldonor gekuppelt Dies können Glycosylhalogenide, -trichloracetimidate oder andere sein.

Als Promotoren für die Glykosidierung eignen sich im allgemeinen Silber, Quecksilber und Ammoniumsalze oder Lewis-Säuren, wie beispielsweise Bortrifluorid-ethyletherat, Trimethysilyltrifluormethansulfonat, Bortribromid oder Aluminiumtrichlorid und bei der Amidkupplung Carbodiimide, wie beispielsweise N,N'- Dicyclohexylcarbodiimid (DCC) oder N-(3-Dimethylaminopropyl)-N'-ethyl-carbodiimid-hydrochlorid (EDC). Bevorzugt sind Bortrifluorid-ethyletherat und EDC.

Als Promotoren für die Umsetzung mit Enolethern eignen sich im allgemeinen substituierte Arylsulfonsäuren, wie beispielsweise Naphthylsulfonsäure, p-Toluolsulfonsäure (PTS) oder Pyridinium-p-toluolsulfonat (PPTS); bevorzugt sind PPTS und PTS.

Als Lösemittel eignen sich organische Lösemittel, die die Löslichkeit des jeweiligen Promotors gewährleisten. Hierzu gehören im allgemeinen Halogenkohlenwasserstoffe wie Methylenchlorid, Chloroform oder Tetrachlorkohlenstoff oder Ether, wie beispielsweise Diethylether, Tetrahydrofuran und Dioxan, Dimethylsulfoxid oder Dimethylformamid.

Im Fall des Promotors Bortriflourid-ethyletherat sind Methylenchlorid und Chloroform, im Fall von EDC sind Dimethylformamid und Dimethylsulfoxid bevorzugt.

Der Promotor wird in einer Menge von 0,2 mol bis 10,0 mol, bevorzugt von 3,0 mol bis 8,0 mol, bezogen auf 1 mol der Verbindungen der allgemeinen Formel (I), (II) oder (III) eingesetzt.

Die Umsetzung wird im allgemeinen bei Normaldruck unter einer Inertgasatmosphäre durchgeführt. Es ist aber auch möglich, das Verfahren bei Überdruck oder bei Unterdruck durchzuführen (z.B. in einem Bereich von 0,5 bis 5 bar).

Das erfindungsgemäße Verfahren wird im allgemeinen in einem Temperaturbereich von -30°C bis +100°C durchgeführt.

Unter Derivatisierungen werden die Deacetylierungen zur Abspaltung der Schutzgruppen am Zucker aufgeführt.

Die Abspaltung der Schutzgruppen am Zuckerrest erfolgt nach üblicher Methode in inerten Lösemitteln in Anwesenheit einer Base oder durch Hydrogenolyse.

Als Basen eignen sich für die Abspaltung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriummethanolat oder Kaliummethanolat eingesetzt.

Als Lösemittel eignen sich für die Abspaltung die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet Ebenso ist es möglich, Gemische der genannten Lösemittel einzusetzen.

Die Abspaltung wird im allgemeinen in einem Temperaturbereich von 0°C bis +100°C, bevorzugt von +20°C bis +60°C durchgeführt. Im allgemeinen wird die Abspaltung bei

Normaldruck durchgeführt. Es ist aber auch möglich, bei Unterdruck oder bei Überdruck zu arbeiten (z.B. von 0,5 bis 5 bar).

Die Abspaltung von speziellen Hydroxyschutzgruppen (Silyl- und Benzylgruppen) erfolgt beispielsweise mit Tetrabutylammoniumfluorid oder anderen Fluoridverbindungen bzw. durch hydrogenolytische Spaltung, im Fall der Benzylgruppe in Anwesenheit eines Katalysators, beispielsweise ein Gemisch aus Palladium/C und Palladium/CaCO₃, mit Wasserstoff oder durch Zusatz von Ammoniumformiat, in einem der oben aufgeführten Lösemittel, vorzugsweise Tetrahydrofuran, Methanol oder Aceton, in einem Temperaturbereich von 0°C bis 60°C, vorzugsweise bei 20°C bis 50°C oder durch Lithium oder Natrium in flüssigem Ammoniak.

Die Abspaltung der Aminoschutzgruppen wird im allgemeinen ebenfalls nach bekannten Methoden, beispielsweise mit Lewissäuren in Dichlormethan, durchgeführt.

Die Verbindungen der allgemeinen Formel (IX), (X) (XI), (XII), (XIII) (XX) und (XXI) sind an sich bekannt und können nach literaturbeschriebenen Verfahren hergestellt werden.

Die Verbindungen der allgemeinen Formel (XIV), (XV), (XVI), (XVII), (XVIII) und (XIX) sind neu und können nach den oben aufgeführten Verfahren hergestellt werden.

Die Glykoside der 6-Hydroxy-2,3-dihydro-1H-indole der allgemeinen Formel (IV), die Glykoside der 5-Hydroxy-1,2-dihydro-3H-pyrrolo[3,2-e]indole (V), die Glykoside der 5-Hydroxy-1,2-dihydro-3H-benzo[e]indole (VI) und die Glykoside der 6-Hydroxy-1,2,3,4-tetrahydro-benzo[*f*]-chinoline der allgemeinen Formeln (VII) sowie die Acetale der 6-Hydroxy-1,2,3,4-tetrahydro-benzo[*f*]-chinoline der allgemeinen Formel (VIII) stellen eine weitgehend untoxische Transportform dieser hochzytociden Verbindungen dar. Durch Verwendung von Glykohydrolasen, die vorzugsweise an tumorspezifische monoklonale Antikörper geknüpft sind, und im Fall von (VIII) durch H⁺ werden die weitgehend untoxischen Transportformen in die hochzytociden 6-Hydroxy-2,3-dihydro-1H-indol- bzw. 5-Hydroxy-1,2-dihydro-3H-pyrrolo[3,2-e]indol- bzw. 5-Hydroxy-1,2-dibydro-3Hbenzo[e]indol-Derivate bzw. 6-Hydroxy-1,2,3,4-tetrahydro-benzo[*f*]-chinoline mit freier phenolischer Hydroxygruppe überführt. Damit ist die Möglichkeit geschaffen, aus einer weitgehend untoxischen Verbindung selektiv im Tumor eine zytocide Verbindung freizusetzen, wobei die Konzentration der zytociden Verbindung im Normalgewebe gering ist Die dosislimitierenden Nebenwirkungen der toxischen 6-Hydroxy-2,3-dihydro-1H-indol- bzw. 5-Hydroxy-1,2-dihydro-3H-pyrrolo[3,2-e]indol- bzw. 5-Hydroxy-1,2-dihydro-3Hbenzo[e]indol-Derivate bzw. 6-Hydroxy-1,2,3,4-tetrahydro-benzo[*f*]-chinoline lassen sich auf diese Weise verringern.

### Untersuchungen zur Zytotoxizität

### Zellinie: A 549

Substanzen: [3-((5'-(((*1H*-*Indol*-2"-yl)-carbonyl)-amino)-1*H*-indol-2'-yl)carbonyl)-1-(1'chlorethyl)-1,2-dihydro-3*H*-benz[e]indol-5-yl]-β-D-galactopyranosid (*seco*-Methyl-CBI-II-Gal) und 2-Chlor-4-[5'-(((1*H*-indol-2"-yl)-carbonyl)-amino)-1*H*-indol-2'-yl)carbonyl]-1,2,3,4-tetrahydro-benzo[*f*]chinolin-6-yl}-β-D-galactopyranosid (7, seco-CBC-II-Gal)
Zellkultur: Die Kultivierung der Zellinie A 549 (ATCC no. CCL 185) in Form von Monolayer-Kulturen erfolgte bei 37°C und 7.5% CO₂ in Luft in DMEM (Dulbecco's Modified Eagle's Medium der Fa. Biochrom, Best-Nr. TO43-10), das mit 10% fetalem Käberserum (Fa. Gibco) supplementiert wurde.
Toxinexposition: Die Verbindungen wurden vor dem Versuch frisch mit DMSO (Fa. Merck, Best.-Nr. 2950.0500) gelöst.

Die Toxinverdünnungen erfolgten für pH 7.4 in DMEM. Der pH-Wert des Kulturmediums wurde vorher mit 0.1 N HCl unter Berücksichtigung der pH-Schwankungen durch die CO₂-Begasung eingestellt. Nachdem die Zellen in den Konzentrationen 10², 10³, 10⁴ und 10⁵ in 6-well Gewebekulturplatten (Fa. Becton Dickinson, Best.-Nr. 3046) eingebracht und bis zur Adhärenz kultiviert wurden, erfolgte die Toxinexposition für 24 h jeweils einmal mit und ohne Zusatz von 0.4 U/ml β-D-Galactosidase. AnschlieBend wurden die Zellen mit frischem Kulturmedium, pH 7.4, versehen und 12 Tage bis zur makroskopischen Koloniebildung kultiviert. Die Kolonien wurden fixiert, mit Löffler's Methylenblau (Fa. Merck, Best.-Nr. 1287) angefärbt und ausgezählt.

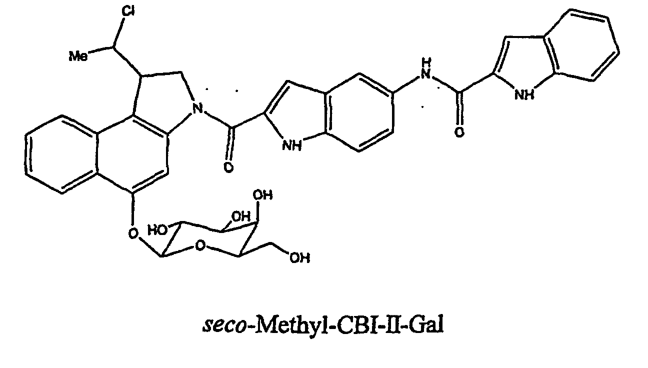

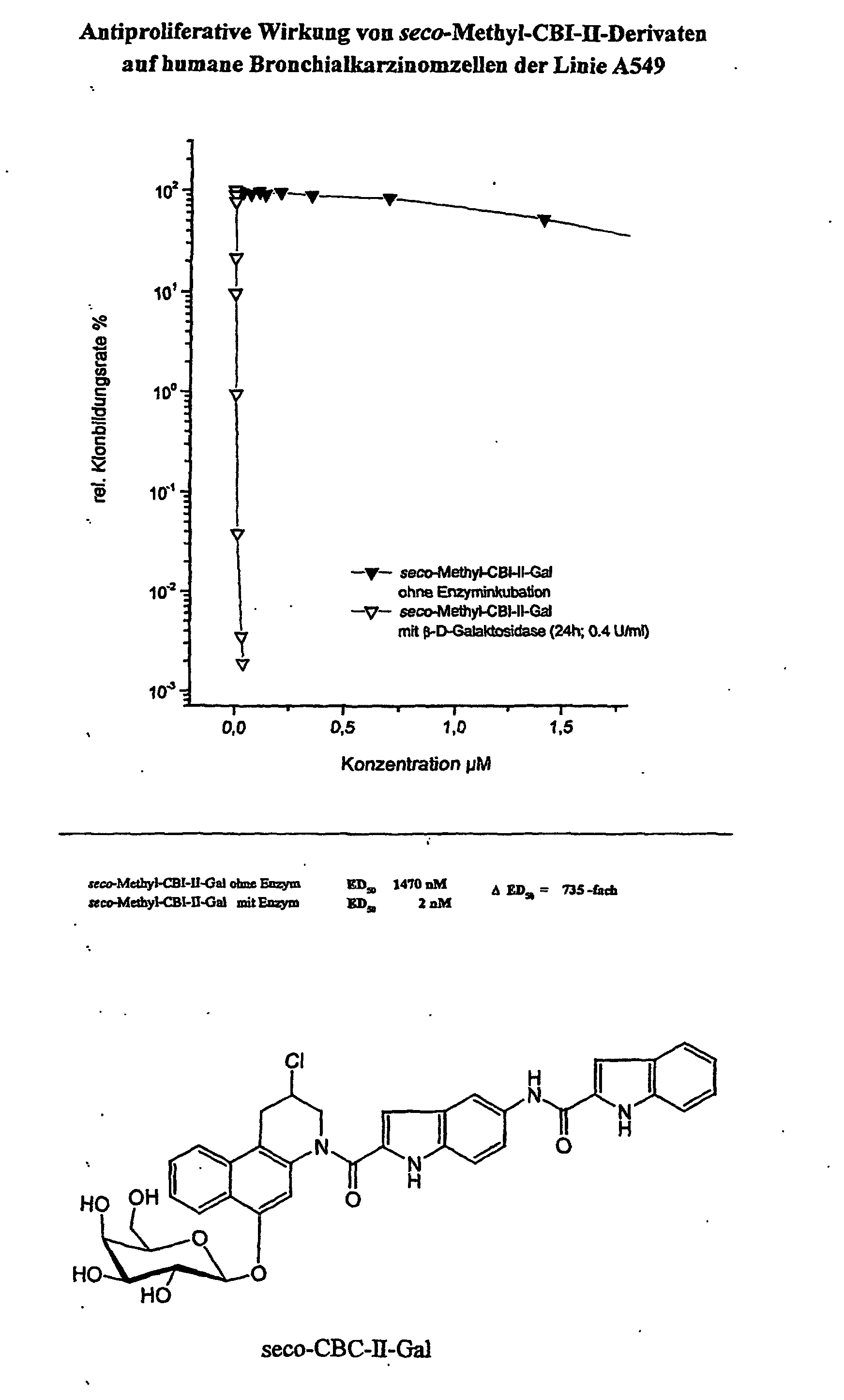

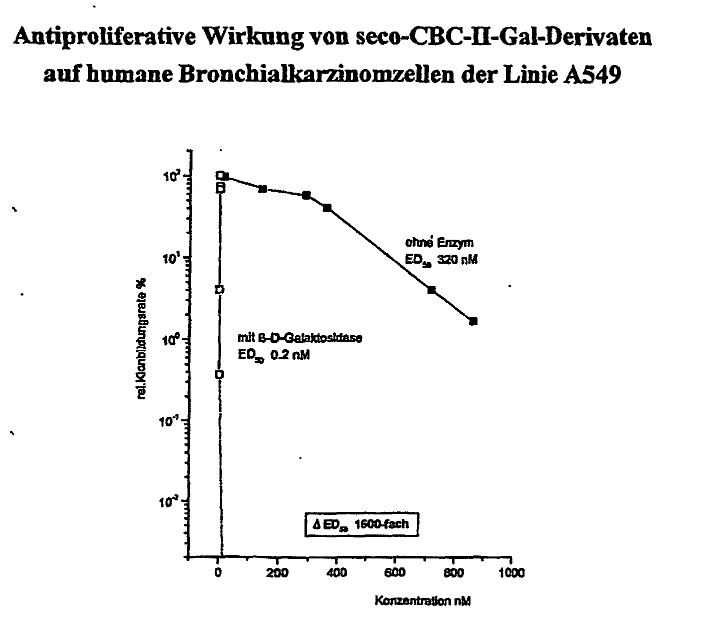

Zur vorliegenden Erfindung gehören pharmazeutische Zubereitungen, die neben nichttoxischen, inerten, pharmazeutisch geeigneten Trägerstoffen eine oder mehrere Verbindungen der Formel (I), (II), (III), (IV), (V) und/oder (VIentbalten oder die aus einem oder mehreren Wirkstoffen der Formel (I), (II), (III), (IV), (V) und/oder (VI), (VII), (VIII) bestehen, sowie Verfahren zur Herstellung dieser Zubereitungen.

Die Wirkstoffe der Formel (I), (II), (III), (IV), (V) und/oder (VI), (sollen in den oben aufgeführten pharmazeutischen Zubereitungen in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I), (II), (III), (IV), (V) und/oder (VI), (VII), (VIII) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 150 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 100, insbesondere 1 bis 80 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

### Experimenteller Teil

### 1. Allgemeine Methoden

Umsetzungen wurden soweit nötig in ausgeheizten Glasapparaturen unter einem leichten Argon-Überdruck durchgeführt. Die Lösungsmittel wurden entsprechend den üblichen Laboratoriumsmethoden getrocknet und destilliert Käufliche Produkte wurden in der Regel ohne weitere Reinigung eingesetzt.

### 1.1 Verwendete Geräte

Schmelzpunkte: Schmelzpunktbestimmungsapparatur FP61 der Firma *Mettler*. Die Werte sind nicht korrigiert.
Infrarotspektren: Modell IFS 25 der Firma *Bruker.* Kristalline Substanzen wurden als KBr-Presslinge, nichtkristalline Verbindungen als Film zwischen KBr-Platten gemessen. Zur Kalibrierung diente die Polystyrolbande bei 1601 cm⁻¹.
UV/VIS-Spektren: Modelle Lambda 2 und Lambda 9 der Firma *Perkin-Elmer*.
¹H-NMR-Spektren: Modell AMX-300 (300 MHz) der Firma *Bruker* und Modell VXR-500 (500 MHz) der Firma *Varian*. Die chemischen Verschiebungen sind in Einheiten der δ-Skala angegeben. Tetramethylsilan (δ_{TMS} = 0.00 ppm) diente als interner Standard. Zur Kennzeichnung der Multiplizitäten der Signale werden folgende Abkürzungen gebraucht: s (Singulett), d (Dublett), t (Triplett), m (Multiplett), m_{c} (zentriertes Multiplett), br (breites Signal). Die Spektren wurden in der Regel erster Ordnung entsprechend interpretiert. Die Kopplungskonstanten *J* sind in Hertz (Hz) angegeben.
¹³C-NMR-Spektren: Modelle XL-200, VXR-200 (50.3 MHz), VXR-500 (125 MHz) der Firma *Varian*, Modell AMX-300 (75.5 MHz) der Firma *Bruker.* Als interner Standard diente Tetramethylsilan oder das angegebene Lösungsmittel. Die chemischen Verschiebungen sind den ¹H-breitbandentkoppelten Spektren entnommen, die Multiplizitäten der Signale wurden in multiplett-selection-Experimenten (APT-Pulsfolge) bestimmt.
Massenspektren: Modelle MAT 311A (niederaufgelöste Spektren)¹ und MAT 731 (hochaufgelöste Spektren) der Firma *Varian*. In Klammern sind die relativen Intensitäten bezogen auf den Basispeak (I = 100) angegeben.
Elementaranalysen: Mikroanalytisches Labor *Hambloch* des Instituts für Organische Chemie der Universität Göttingen.

### 1.2 Chromatographische Methoden

Dünnschichtchromatographie (DC): Es wurden DC-Fertigfolien SIL G/UV₂₅₄ der Firma *Machery*, *Nagel & Co.* (Schichtdicke 0.25 mm) verwendet. Angegeben sind R_{f}-Werte (Laufhöhe relativ zur Laufmittelfront). Als Abkürzungen für die verwendeten Lösungsmittel werden benutzt: EE (Essigester), PE (Petrolether des Siedebereiches 40-75 °C, CH₂Cl₂ (Dichlormethan). Neben der UV-Detektion diente eine Vanillin-Schwefelsäure-Lösung (0.5 g Vanillin, 3 ml Schwefelsäure, 85 ml Methanol und 10 ml Essigsäure) als Anfärbereagenz.

Säulenfiltration (SF) und Säulenchromatographie (SC): Alle säulenchromatogra-phischen Trennungen wurden mit Kieselgel 60 (Korngröße: 0.063-0.200 mm) der Firma *Machery, Nagel & Co.* oder mit Kieselgel 60 (Korngröße: 0.200-0.400 mm) durchgeführt. In Abhängigkeit vom Trennproblem verwendete man ein Substanz/Absorbens-Verhältnis zwischen 50:1 und 200:1.

### Erläuterung zu den Abkürzungen im experimentellen Teil:

- PE =: Petrolether
- Lsg. =: Lösung
- EtOAc =: Essigsäureethylester
- DMF =: Dimethylformamid
- EDC =: N-(3-Dimethylaminopropyl)-N'-ethyl-carbodiimid-hydrochlorid
- AIBN =: α,α'-Azo-isobutyronitril

### Herstellungsbeispiel

### Beispiel 1: 2-Amino-4-benzyloxy-N-[E/Z-1'-(3'-chlor)-but-2'-enyl]-N-(tert.butoxycarbonyl)-1-iod-naphthalin

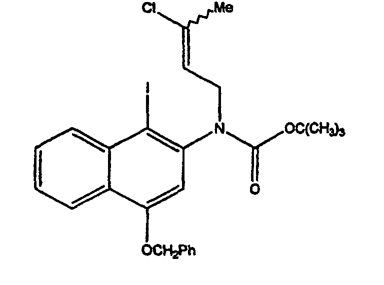

Zu einer Lösung von 2.00 g (4.21 mmol) 2-Amino-4-benzyloxy-N-(*tert*.-butoxycarbonyl)-1-iod-naphthalin [herstellbar beispielsweise nach D. L. Boger, J. A. McKie, J. Org. Chem. 1995, 60,1271] in 50.0 ml trockenem DMF wurden 400 mg (10.0 mmol) Natriumhydrid als 60%ige Suspension in Paraffinöl hinzugefügt. Man rührte 45 min bei Raumtemperatur und tropfte dann 1.20 ml (1:37 g, 10.9 mmol) eines Isomerengemisches aus E/Z-1,3-Dichlor-but-2-en zu. Anschließend wurde 3 h bei Raumtemperatur gerührt. Nach Hydrolyse mit ges. NH₄Cl-Lösung wurde dreimal mit EtOAc extrahiert. Die vereinigten organischen Phasen wurden fünfmal mit Wasser und danach einmal mit ges. NaCl-Lsg. gewaschen und über Na₂SO₄ getrocknet. Die Lösemittel wurden im Vakuum entfernt; eine säulenchromatographische Reinigung des Rückstandes (100 g Kieselgel, 40-63 µm, Laufmittel PE/EtOAc 10:1) ergab 2.32 g (4.11 mmol, 98% Ausbeute) der Zielverbindung als leicht gelbliches Öl.
R_{f}= 0.40 (E) bzw. 0.33 (Z) (PE/EtOAc 10:1)

### Beispiel 2: 5-Benzyloxy-3-(tert.-butoxycarbonyl)-1-(1'-chlorethyl)-1,2-dihydro-3Hbenzo[e]indol

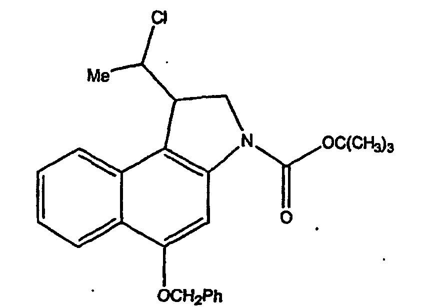

573 mg (1.02 mmol) der Verbindung aus Beispiel 1 wurden in 18 ml trockenem, entgastem Toluol gelöst und mit 0.35 ml (384 mg, 1.32 mmol) Tributylzinnhydrid und 42.0 mg (255 µmol) AIBN versetzt. Das Gemisch wurde auf 80°C erhitzt und 3.5 h bei dieser Temperatur gerührt. Der nach dem Einengen erhaltene Rückstand wurde in Diethylether aufgenommen und mit dem gleichen Volumen einer 10%igen wäßrigen Lsg. von KF gewaschen. Der nach dem Trocknen der org. Phase über Na₂SO₄ und dem Entfernen der Lösemittel erhaltene Rückstand wurde einer säulenchromatographischen Reinigung (100 g Kieselgel, 40 - 63 µm, Laufmittel PE/EtOAc 20:1) unterworfen. Man erhielt 187 mg (419 µmol, 42% Ausbeute) des syn- und 180 mg (411 µmol, 41% Ausbeute) des anti-Diastereomers der Zielverbindung.
R_{f}= 0.52 (syn) bzw. 0.32 (anti) (PE/EtOAc 10:1)

### Beispiel 3: syn-3-(tert.-Butoxycarbonyl)-1-(1'-chlorethyl)-5-hydroxy-1,2-dihydro-3Hbenzo[e]indol

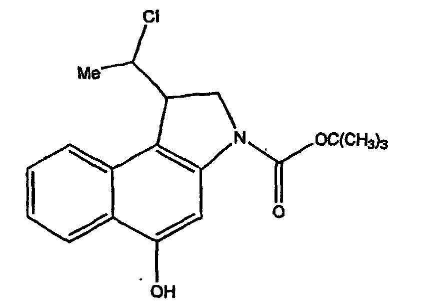

354 mg (808 µmol) des syn-Isomers der Verbindung aus Beispiel 2 wurden in 17.0 ml Aceton gelöst und mit 389 mg (366 µmol) 10% Pd auf Aktivkohle sowie 318 mg (5.05 mmol) Ammoniumformiat versetzt. Man ließ 2 h bei 50°C rühren. Die Reaktionslösung wurde über Celite filtriert, welches mit EtOAc gründlich gewaschen wurde. Der nach Einengen des Filtrats erhaltene Rückstand wurde durch Säulenchromatographie an Kieselgel (40 g, 40 - 63 µm, Laufmittel PE/EtOAc 5:1) gereinigt und ergab somit 111 mg (320 µmol, 85% Ausbeute) der Zielverbindung.
R_{f}= 0.48 (PE/EtOAc 5:1)

### Beispiel 4: syn-[3-((5'-(((1H-Indol-2"-yl)carbonyl)amino)-1H-indol-2'-yl)carbonyl)-1-(1'chlorethyl)-1,2-dihydro-3H-benzo[e]indol-5-yl]-2,3,4,6-tetra-O-acetyl-β-D-galactopyranosid

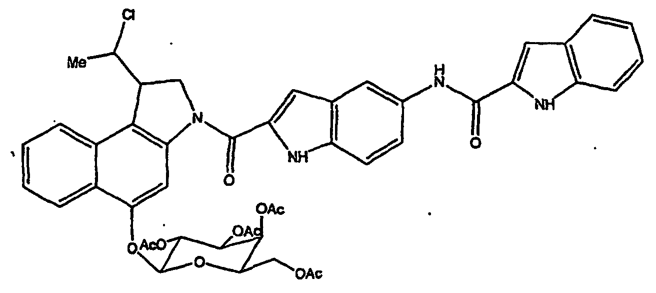

50.0 mg (144 µmol) der Verbindung aus Beispiel 3 wurden in 7.0 ml trockenem Dichlormethan gelöst. Dazu gab man 1.00 g gründlich ausgeheiztes Molsieb (4Å) sowie 74.0 mg (148µmol) O-(2,3,4,6-Tetra-O-acetyl-α-D-galactopyranosyl)-trichloracetimidat. Man rührte 30 min bei Raumtemperatur und gab dann bei -10°C langsam 59.0 µl (66.7 mg, 470 µmol) Bortrifluorid-ethyletherat zu. Man rührte noch 1 h bei -10°C, anschließend 4 h bei Raumtemperatur. Das Gemisch wurde im Hochvakuum bis zur Trockenheit eingeengt und dann unter einer Argon-Atmosphäre in 2.5 ml trockenem, entgastem DMF suspendiert. Man versetzte mit 40.9 mg (128µmol) 5-[((1H-Indol-2'-yl)carbonyl)amino]-1H-indol-2-carbonsäure sowie 62.0 mg (320 µmol) EDC und ließ 36 h bei Raumtemperatur rühren. Das Gemisch wurde bis zur Trockenheit eingeengt und der Rückstand einer Säulenchromatographie an Kieselgel (50 g, 40 - 63 µm, Laufmittel 33-66% EtOAc in PE) unterworfen. Man erhielt 69.5 mg (79.0 µmol, 55% Ausbeute) der Zielverbindung.
R_{f}= 0,58 (PE/Aceton/Methanol 10:6:1)

### Beispiel 5: syn-[3-((5'-(((1H-Indol-2"-yl)carbonyl)amino)-1H-indol-2'-yl)carbonyl)-1-(1'-Chlorethyl)-1,2-dihydro-3H-benzo[e]indol-5-yl]-β-D-galactopyranosid

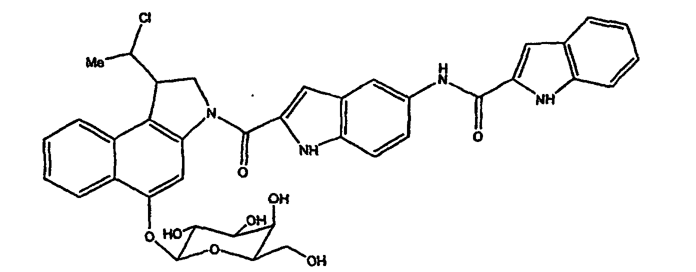

64,0 mg (72.5 µmol) der Verbindung aus Beispiel 4 wurden in 2.0 ml trockenem Methanol gelöst und mit 9.0 µl (48.6 µmol) einer 5.4 M Lsg. von Natriummethanolat in Methanol versetzt. Nach 3 h Rühren bei Raumtemperatur wurden 0.5 ml Wasser zugesetzt und der ausgefallene Niederschlag abfiltriert. Man erhielt 19.2 mg (27.0 µmol, 37% Ausbeute) der Zielverbindung:

### Beispiel 6: Alkohol 1 ist literaturbekannt (D. L. Boger, J. A. McKie, C. W. Boyce, Synlett 1997, 515-517) und wurde in einer leicht modifizierten Synthesesequenz nach laboratoriumsüblichen Verfahren hergestellt.

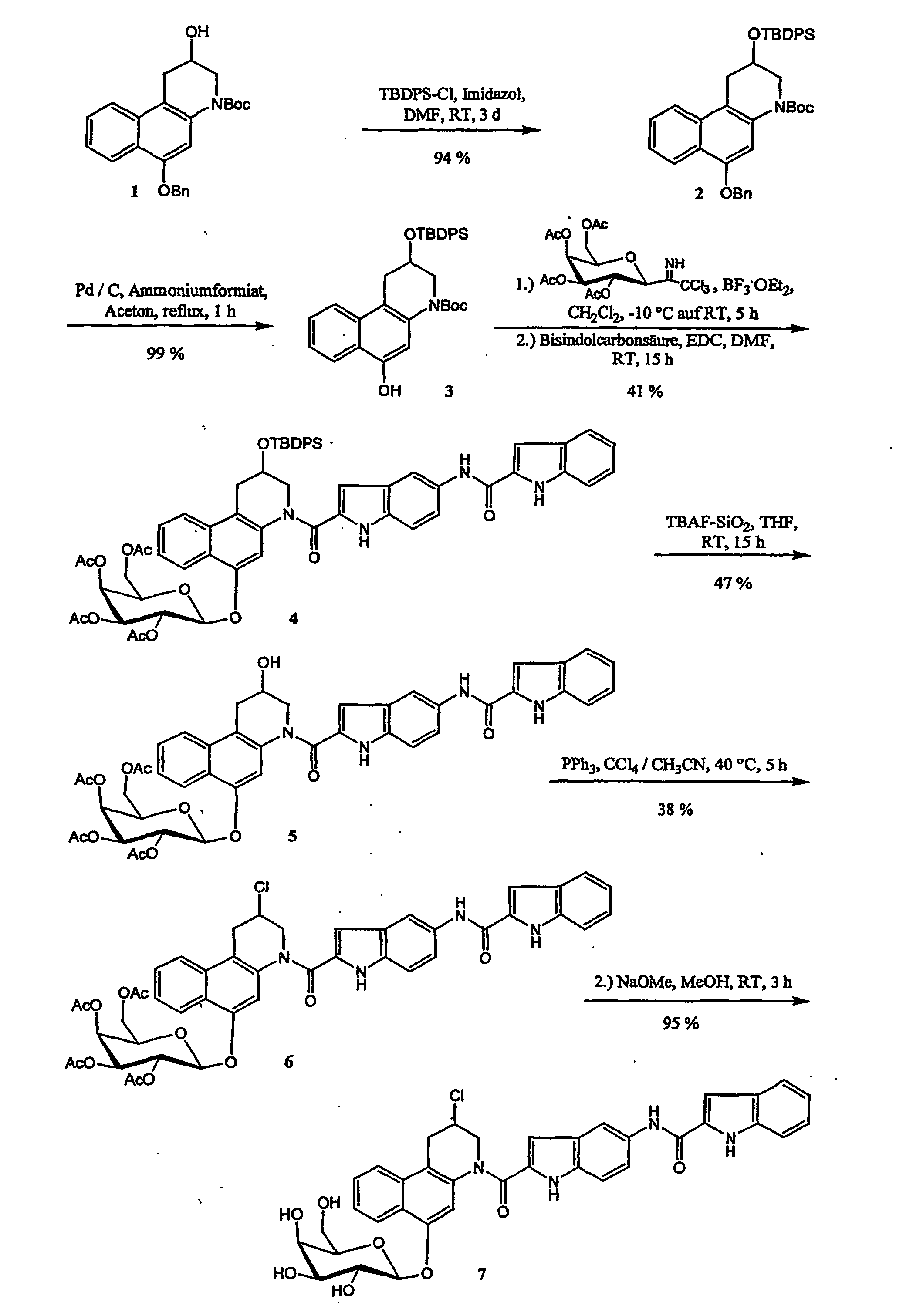

### 6-Benzyloxy-2-(tert-butyl-diphenyl-silanyloxy)-N-(tert-butoxycarbonyl)-2,3-dihydro-1Hbenzo[f]chinolin (2):

Zu einer Mischung aus Imidazol (1.47 g, 21.6 mmol, 5 Äq.) und *tert-*Butyldiphenylsilylchlorid (2.21 ml, 2.37 g, 8.63 mmol, 2 Äq.) gab man eine Lösung des Alkohols 1 in absolutem DMF (5 ml) und rührte das Reaktionsgemisch drei Tage bei Raumtemperatur. Zur Aufarbeitung wurde die gelbe Lösung auf Eiswasser gegeben, zweimal mit CH₂Cl₂ und einmal mit Et₂O extrahiert. Die vereinigten organischen Phasen wurden dreimal mit ges. Zitronensäurelösung und abschließend mit ges. NaCl-Lösung gewaschen, über MgSO₄ getrocknet und das Lösungsmittel im Vakuum entfernt. Das so erhaltene ölige gelbe Rohprodukt lieferte nach säulenchromatographischer Reinigung (PE / EE = 30 : 1) 2.60 g (94%) Silylether **2** in Form eines weißen festen Schaums.
R_{f} = 0.59 (PE / EE = 3 : 1), braun (VSS);
UV (CH₃CN): λₘₐₓ (lg ε) = 253 nm (3.859), 303 (3.089)
IR (KBr): 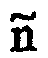 = 3070 cm⁻¹ (Ar-H), 2930 (CH), 1702 (C=O), 1595, 1367, 1254, 1158, 757.
¹H-NMR (300 MHz, CDCl₃): δ = 1.08 (s, 9 H, SiC(CH₃)₃), 1.46 (s, 9 H, OC(CH₃)₃), 2.90 (dd, *J=* 17.0, 6.4 Hz, 1 H, 1-Hₐ), 3.05 (dd, *J*= 17.0, 6.0 Hz, 1 H, 1-H_{b}), 3.62 (dd, *J=* 12.5, 8.3, Hz, 1 H, 3-Hₐ), 4.00 (dd, *J*= 12.5, 3.0 Hz, 1 H, 3-H_{b}), 4.29 (dddd, *J*= 8.3, 6.4, 6.0, 3.0 Hz, 1 H, 2-H), 5.19 (s, 2 H, C*H*₂Ph), 7.22 (s, 1 H, 5-H), 7.32 - 7.48 (m, 11 H, 5 × Bn-H, 4 × Ph-H_{*m*}, 2 × Ph-H_{*p*}), 7.52 - 7.54 (m, 2 H, 8-H, 9-H), 7.59 (d, *J*= 8.3 Hz, 1 H, 10-H), 7.70 - 7.75 (m, 4 H, 4 × Ph-H_{*o*}), 8.29 (dd, *J*= 8.3, 1.1 Hz, H, 7-H).
¹³C-NMR (50 MHz, CDCl₃): δ = 19.22 (SiC(CH₃)₃), 26.98 (SiC(CH₃)₃), 28.39 (OC(CH₃)₃), 33.76 (C-1), 50.24 (C-3), 66.69 (C-2), 70.10 (CH₂Ph), 80.93 (OC(CH₃)₃), 103.8 (C-5), 113.7 (C-10b), 122.3, 122.4, 124.1 (C-7, C-10, C-8), 123.4 (C-6a), 127.8 (C-9), 127.6 (2 × Bn-C_{*o*}), 127.7 (4 × Ph-C_{*m*}), 127.9 (Ph-C_{*p*}), 128.5 (2 × Bn-C_{*m*}), 129.8 (2 × Ph-C_{*p*}), 132.6 (C-10a), 133.7,134.0 (2 × Ph-C_{*i*}), 135.7 (4 × Ph-C_{*o*}), 135.8 (Bn-C_{*i*}), 137.1 (C-4a), 152.3 (C=O), 154.9 (C-6).
MS (DCI, NH₃); *m*/*z* (%) = 661 (70) [M+NH₄]⁺, 644 (12) [M+H]⁺, 605 (22) 274 (100).

| | | | | |
|---|---|---|---|---|
| C₂₅H₂₇NO₄ (387.47). | Ber. | C:76.48 | H: 7.05 | N: 2.18 |
| | Gef. | C: 76.35 | H: 7.29 | N: 2.12 |

### 2-(tert-Butyldiphenylsilanyloxy)-6-hydroxy-4-(tert-butoxycarbonyl)-2,3-dihydro-1Hbenzo[f]chinolin (3):

Eine Lösung des Benzylethers 2 (2.60 g, 4.04 mmol) in Aceton (100 ml) wurde mit Ammoniumformiat (1.65 g, 26.3 mmol, 6.5 Äq.) und 10% Pd auf Aktivkohle (2.15 g, 2.02 mmol, 0.5 Äq.) versetzt und eine Stunde refluxiert. Nach Abkühlung auf Raumtemperatur wurde der Katalysator über Celite abfiltriert und gründlich mit Aceton nachgewaschen. Nach Entfernen des Lösungsmittels im Vakuum und Säulenfiltration (PE / EE = 5 : 1) erhielt man 2.22 g (99 %) eines weißen Schaums.
Smp.: 71 - 73 °C
R_{f} = 0.40 (PE / EE = 5 : 1), gelb (VSS);
UV (CH₃CN): λₘₐₓ (lg ε) = 253 nm (3.842), 299 (2.986), 271 (2.777).
IR (KBr): 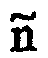 = 3372 cm⁻¹ (OH), 3070 (Ar-H), 2931(CH), 1675 (C=O), 1597, 1368, 1256, 1156, 758.
¹H-NMR (300 MHz, CDCl₃): δ = 1.03 (s, 9 H, SiC(CH₃)₃), 1.37 (s, 9 H, OC(CH₃)₃), 2.83 (dd, *J*= 16.6, 6.8 Hz, 1 H, 1-Hₐ), 2.99 (dd, *J*= 16.6,6.4 Hz, 1 H, 1-H_{b}), 3.49 (dd, *J*= 12.4, 8.3 Hz, 1 H, 3-Hₐ), 3.94 (dd, *J*= 12.4, 3.4 Hz, 1 H, 3-H_{b}), 4.29 (dddd, *J*= 8.3, 6.8, 6.4, 3.4 Hz, 1 H, 2-H), 7.01 (s_{br}, 1 H, 6-OH), 7.14 - 7.39 (m, 9 H, 5-H, 8-H, 9-H, 4 × Ph-H_{*m*}, 2 × Ph-H_{*p*}), 7.46 (d, *J*= 8.3 Hz, 1 H, 10-H), 7.62 - 7.67 (m, 4 H, Ph-H_{*o*}), 7.97 (d, *J*= 7.5 Hz, 1 H, 7-H). ¹³C-NMR (50 MHz, CDCl₃): δ = 19.26 (SiC(CH₃)₃), 27.00 (SiC(CH₃)₃), 28.27 (OC(CH₃)₃), 33.71 (C-1), 50.40 (C-3), 66.69 (C-2), 81.37 (OC(CH₃)₃), 105.7 (C-5), 112.9 (C-10b), 122.2, 122.5,123.7 (C-7, C-10, C-8), 122.7 (C-6a), 126.5 (C-9), 127.7 (4 × Ph-C_{*m*}), 129.8 (2 × Ph-Cₚ), 132.7 (C-10a), 133.8, 134.0 (2 × Ph-C_{*t*}), 135.4 (C-4a), 135.7 (4 × Ph-C_{*o*}), 150.4 (C=O), 154.3 (C-6).
MS (DCI, NH₃): *m*/*z* (%) = 571. (100) [M+NH₄]⁺.

| | | | |
|---|---|---|---|
| C₃₄H₃₉NO₄Si (553.76). | Ber. | C:73.74 | H: 7.10 |
| | Gef. | C: 73.53 | H: 7.32 |

### {4-[5'-((1H-Indol-2"-carbonyl)-amino)-1H-indol-2'-carbonyl]-2-(tert-butyldiphenylsilanyloxy)-1,2,3,4-tetrahydro-benzo[f]chinolin-6-yl}-2*,3*,4*,6*-tetra-Oacetyl-β-D-galactopyranosid (4):

Phenol **3** (500 mg, 903 µmol) und *O*-(2,3,4,6-Tetra-*O*-acetyl-α-D-galactopyranosyl)-trichloracetimidat (490 mg, 993 µmol, 1.1 Äq.) wurden in absolutem CH₂Cl₂ (50 ml) gelöst, ca. 30 min über Molsieb 4Å gerührt und dabei auf -10 °C gekühlt. Bei dieser Temperatur wurde BF₃•OEt₂ (56.7 µl, 64.1 mg, 451 µmol, 0.5 Äq.) zugetropft, wobei sofortige Gelbfärbung auftrat. Nach dreistündigem Rühren bei -10 °C gab man weiteres BF₃•OEt₂ (340 µl, 384 mg, 2.71 mmol, 3.0 Äq.) zu und ließ auf Raumtemperatur erwärmen. Fünf Stunden später wurde das Reaktionsgemisch via Transferkanüle in einen zweiten Kolben überführt und so vom Molsieb abgetrennt. Der nach Entfernen des Lösungsmittels durch Umkondensieren zurückgebliebene gelbe Feststoff wurde ca. eine Stunde im Vakuum getrocknet und anschließend in absolutem DMF (20 ml) aufgenommen. Nach Zugabe von Bisindolcarbonsäure (288 mg, 903 µmol, 1.0 Äq.) und EDC (519 mg, 2.71 mmol, 3.0 Äq.) wurde 14 Stunden bei Raumtemperatur gerührt und der dabei entstandene gelbe Niederschlag anschließend über wenig Celite abfiltriert (Nachspülen mit CH₂Cl₂). Das Filtrat wurde mit Wasser und ges. NaCl-Lösung gewaschen, über MgSO₄ getrocknet und das Lösungsmittel im Vakuum entfernt. Nach säulenchromatographischer Aufreinigung mit PE / EE = 3 : 2 erhielt man 406 mg (41 %) des gewünschten Produktes (**4**) als gelben Feststoff zusammen mit 120 mg (15 %) freiem Amin.
R_{f}= 0.14 (PE / EE = 3 : 2), rot (VSS);
UV (CH₃CN): λₘₐₓ (lg ε) = 311 nm (3.588).
IR (KBr): 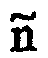 = 3405 cm⁻¹ (NH), 3071 (Ar-H), 2932 (CH), 1753 (C=O), 1620 (C=C), 1597, 1370, 1229, 1078, 745.
¹H-NMR (300 MHz, Aceton-d₆): δ = 1.00 (s, 9 H, C(CH₃)₃), 1.89, 1.95, 2.11 (3 × s, 12 H, 4 × C(O)CH₃), 3.13 - 3.22 (m, 1 H, 1-Hₐ), 3.29 (dd, *J*= 17.3, 5.3 Hz, 1 H, 1-H_{b}), 3.58 - 3.69 (m, 1 H, 3-Hₐ), 3.82 - 4.07 (m, 3 H, 6*-H₂, 5*-H), 4.09 - 4.30 (m, 1 H, 3-H_{b}), 4.51 - 4.58 (m, 1 H, 2-H), 4.70 (d, *J=* 8.0 Hz, 1 H, 1*-H), 4.87 (dd, *J=* 10.5, 3.4 Hz, 1 H, 3*-H), 5.31 (dd, *J*= 7.5, 3.4 Hz, 1 H, 4*-H), 5.40 (dd, *J*= 10.5, 8.0 Hz, 1 H, 2*-H), 6.68 (s, 1 H, 3'-H), 7.01 - 7.04 (m, 1 H, 5"-H), 7.06 (s, 1 H, 5-H), 7.18 - 7.69 (m, 18 H, Ph-H, 3"-H, 4"-H, 6"-H, 7"-H, 6'-H, 7'-H, 8-H, 9-H), 7.80 (dd, *J*= 8.9, 8.9 Hz, 1 H, 10-H), 7.99 - 8.04 (m, 1 H, 7-H), 8.24 (s, 1 H, 4'-H), 9.46 (s, 1H, 5'-NH), 10.70 (s, 1 H, Indol-NH), 10.91 (s, 1H, Indol-NH).
¹³C-NMR (50 MHz, Aceton-d₆): δ = 15.56 (C(CH₃)₃), 20.44 (3 × CHOC(O)CH₃), 20.70 (CH₂OC(O)CH₃), 27.30 (C(CH₃)₃), 33.39 (C-1), 51.56 (C-3), 61.96 (C-6*), 67.20 (C-2), 67.92 (C-4*), 69.01 (C-2*), 71.36 (C-3*), 71.63 (C-5*), 101.3 (C-1*), 103.7 (C-3"), 108.0 (C-3'), 109.2 (C-5), 113.0, 113.1 (C-6', C-7'), 113.9 (C-4'), 116.9 (C-10b), 120.1 (C-5"), 120.9, 125.9,128.0,130.6 (C-7, C-10, C-4", C-7"), 122.5, 123.6 (C-8, C-9), 124.6 (C-6a), 124.7 (C-6"), 128.2, 128.7 (C-3'a, C-3"a), 128.5 (4 × Ph-C_{*m*}, 2 × Ph-C_{*p*}), 132.5, 132.9, 133.0, 133.7, 134.1, 134.4 (C-10a, 2 × Ph-C_{*i*}, C-5', C-2', C-2", C-7'a), 136.5 (4 × Ph-C_{*o*}), 136.8, 137.9 (C-7"a, C-4a), 151.7 (C-6), 160.6 (2'-C=O), 164.1 (2"-C=O), 170.2, 170.6, 170.7, 170.9 (4 × C(O)-CH₃).
MS (DCI, NH₃): *m*/*z* (%) = 1102 (100) [M+NH₄]⁺, 1085 (38) [M+H]⁺.

| | | | | |
|---|---|---|---|---|
| C₆₁H₆₀N₄O₁₃Si (1085.23). | Ber. | C: 67.51 | H: 5.57 | N: 5.16 |
| | Gef. | C: 67.69 | H: 5.45 | N: 5.05 |

### 4-[5'-((1H-Indol-2"-carbonyl)-amino)-1H-indol-2'-carbonyl]-2-hydroxy-1,2,3,4-tetrahydrobenzo[f]chinolin-6-yl}-2*,3*,4*,6*-tetra-O-acetyl-β-D-galactopyranosid (5):

Der Silylether **4'** (300 mg, 276 µmol) wurde in absolutem THF (10 ml) gelöst, mit TBAF auf Kieselgel (754 mg, 829 µmol, 3 Äq.) versetzt und sechs Stunden bei Raumtemperatur gerührt. Zur Aufarbeitung wurden drei kleine Spatel Kieselgel zugegeben und das Lösungsmittel im Vakuum entfernt. Der Rückstand wurde mit PE / EE = 1 : 1 → 1 : 5 säulenchromatographisch gereinigt und lieferte 111 mg (47 %) reinen Alkohol 5 als gelblichen Feststoff.
R_{f}= 0.33 (PE / EE = 1 : 5)
UV (CH₃CN): λₘₐₓ (lg ε) = 214 nm (3.772), 240 (3.638), 310 (3.630).
IR (KBr): 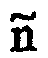 = 3396 cm⁻¹ (NH), 3283 (OH), 3075 (Ar-H), 2932 (CH), 1752 (C=O), 1619 (C=C), 1597, 1370, 1230, 1075, 750.
¹H-NMR (300 MHz, Aceton-d₆): δ = 1.88, 1.94, 1.95, 2.10 (4 × s, 12 H, 4 × C(O)CH₃), 3.09 (dd, *J*= 17.0, 6.8 Hz, 1 H, 1-Hₐ), 3.51 (dd, *J*= 17.0,6.4 Hz, 1 H, 1-H_{b}), 3.62 - 3.72 (m, 2 H, 5*-H, 3-Hₐ), 3.92 - 4.03 (m, 2 H, 6*-H₂), 4.40 (d, *J*= 8.0 Hz, 1 H, 1*-H), 4.43 - 4.51 (m, 1 H, 2-H), 4.54 (d, *J*= 4.2 Hz, 1 H, OH), 4.56 (dd, *J=* 14.3, 3.4 Hz, 1 H, 3-H_{b}), 4.72 (dd, *J*= 10.6, 3.4 Hz, 1 H, 3*-H), 5.29 (dd, *J*= 6.8, 3.4 Hz, 1 H, 4*-H), 5.33 (dd, *J*= 10.6, 8.0 Hz, 1 H, 2*-H), 6.73 (d, *J*= 1.1 Hz, 1 H, 3'-H), 6.86 (s, 1 H, 5-H), 7.07 (ddd, *J*= 7.9, 7.9, 1.1 Hz, 1 H, 5"-H), 7.23 (ddd, *J*= 7.9, 7.9, 1.1 Hz, 1 H, 6"-H), 7.30 (s, 1 H, 3"-H), 7.45 - 7.66 (m, 6 H, 4"-H, 7"-H, 6'-H, 7'-H, 8-H, 9-H), 7.95 - 8.02 (m, 2 H, 7-H, 10-H), 8.24 (d, *J*= 1.1 Hz, 1 H, 4'-H), 9.38 (s_{br}, 1H, 5'-NH), 10.70 (s, 1 H, Indol-NH), 10.83 (s, 1H, Indol-NH).
¹³C-NMR, (75 MHz, Aceton-d₆): δ = 20.53 (3 × CHOC(O)CH₃), 20.71 (CH₂OC(O)CH₃), 33.86 (C-1), 51.66 (C-3), 61.99 (C-6*), 65.72 (C-2), 67.89 (C-4*), 69.01 (C-2*), 71.40 (C-3*), 71.59 (C-5*), 101.6 (C-1*), 108.0 (C-3"), 109.1 (C-3'), 113.0 (C-5), 113.1, 113.2 (C-6', C-7'), 113.9 (C-4'), 118.1 (C-10b), 120.1, 120.9, 125.9, 128.1 (C-8, C-9, C-4", C 7"), 120.2 (C-5"), 122.6, 123.9 (C-7, C-10), 124.5 (C-6a), 124.7 (C-6"), 128.7, 128.8 (C-3'a, C-3"a), 132.9, 133.0, 133.1, 133.8, 134.5, 136.8, 137.8 (C-10a, C-5', C-2', C-2", C-7'a, C-7"a, C-4a), 151.9 (C-6), 160.5 (2'-C=O), 163.8 (2"-C=O), 170.1, 170.3, 170.6, 170.7 (4 × C(O)-CH₃).
MS (ESI): *m*/*z* (%) = 869 (100) [M+Na]⁺, 1715 (33) [2M+Na]⁺.

| | | | | |
|---|---|---|---|---|
| C₄₅H₄₂N₄O₁₃ (846.84). | Ber. | C: 63.81 | H: 5.00 | N: 6.62 |
| | Gef. | C: 63.53 | H: 5.27 | N: 6.46 |

{2-Chlor-4-[5'-((1*H*-indol-2"-carbonyl)-amino)-1*H*-indol-2'-carbonyl]-1,2,3,4-tetrahydrobenzo[*f*]chinolin-6-yl}-2*,3*,4*,6*-tetra-*O*-acetyl-β-D-galactopyranosid **(6):**
Zu einer Lösung des Alkohols **5** (300 mg, 354 µmol) in einem Gemisch aus absolutem CCl₄ und CH₃CN 1 : 1 (12 ml) gab man festes Triphenylphosphan (557 mg, 2.13 mmol, 6 Äq.) zu und rührte das Reaktionsgemisch vier Stunden bei 40 °C. Nach Zugabe von ca. drei Spateln Kieselgel wurde das Lösungsmittel im Vakuum entfernt und der Rückstand mit Toluol / Aceton = 2 : 1 chromatographiert. Man erhielt so insgesamt 226 mg (74 %) eines Gemisches aus dem gewünschten Chlorid **6** und dem entsprechenden Eliminierungsprodukt (Verhältnis ca. 2: 1 ), woraus nach erneuter Chromatographie (PE / EE = 1 : 1 → 3 : 1) 116 mg (38 %) diastereomerenreines Chlorid **6a** als hellgelber Feststoff isoliert werden konnten. Die vereinigten Mischfraktionen bestehend aus dem zweiten Diastereomer und dem entsprechenden Eliminierungsprodukt wurden durch semipräparative HPLC getrennt (Bedingungen siehe unten). Die so erhaltenen Produktfraktionen wurden mit Wasser gesättigt, dreimal mit CH₂Cl₂ extrahiert und die vereinigten organischen Phasen schließlich im Vakuum eingeengt. Eventuell vorhandene Wasserspuren wurden durch azeotrope Destillation mit Benzol entfernt

### Analytische Daten für das erste Diastereomer 6a:

R_{f}= 0.13 (PE / EE = 1:1).
UV (CH₃CN): λₘₐₓ (lg ε) = 213 nm (3.804), 239 (3.695), 311 (3.673).
**IR** (KBr): 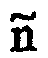 = 3348 cm⁻¹ (NH), 3074 (Ar-H), 2934 (CH), 1752 (C=O), 1621 (C=C), 1598, 1370, 1230, 747.
¹H-NMR (300 MHz, Aceton-d₆): δ = 1.94, 1.96, 2.00, 2.15 (4 × s, 12 H, 4 × C(O)CH₃), 3.47-3.56 (m, 2H, 1-H₂), 3.76-4.10 (m, 3 H, 5*-H, 6*-H₂), 4.14 (dd, *J*= 12.8, 7.2 Hz, 1 H, 3-Hₐ), 4.59 (d, *J*= 8,3 Hz, 1 H, 1*-H), 4.67 (dd, *J*= 12.8, 2.6 Hz, 1 H, 3-H_{b}), 4.85 (dd, *J*= 10.5, 3.4 Hz, 1 H, 3*-H), 4.49-5.02 (m, 1 H, 2-H), 5.35 (d, *J*= 3.0 Hz, 1 H, 4*-H), 5.41 (dd, *J*= 10.5, 8.3 Hz, 1 H, 2*-H), 6.80 (s, 1 H, 3'-H), 6.97 (s, 1 H, 5-H), 7.11 (ddd, *J*= 7.9, 7.9, 1.1 Hz, 1 H, 5"-H), 726 (ddd, *J*= 7.2, 7.2, 1.1 Hz, 1 H, 6"-H), 7.37 (s, 1 H, 3"-H), 7.53-7.71 (m, 6 H, 4"-H, 7"-H, 6'-H, 7'-H, 8-H, 9-H), 8.01-8.08 (m, 2 H, 7-H, 10 H), 8.31 (d, *J*= 1.9 Hz, 1 H, 4'-H), 9.52 (s, 1H, 5'-NH), 10.92 (s, 1 H, Indol-NH), 10.96 (s, 1H, Indol-NH). ¹³C-NMR (75 MHz, Aceton-d₆): δ = 20.53 (3 × CHOC(O)CH₃), 20.70 (CH₂OC(O)CH₃), 35.02 (C-1), 51.37 (C-3), 54.85 (C-2), 61.84 (C-6*), 67.83 (C-4*), 69.05 (C-2*), 71.36 (C-3*), 71.59 (C-5*), 101.5 (C-1*), 103.7 (C-3"), 108.4 (C-3'), 109.1 (C-5), 113.1, 113.2 (C-6', C-7'), 113.9 (C-4'), 116.3 (C-10b), 120.4, 120.9, 122.6, 122.7, 123.7, 124.7, 126.1, 128.4 (C-7, C-8, C-9, C-10, C-4", C-5", C-6", C-7"), 128.1 (C-6a), 128.7, 128.8 (C-3'a, C-3"a), 132.4, 132.9, 133.1, 133.4, 134.6, 136.4, 137.8 (C-10a, C-5', C-2', C-2", C-7'a, C-7"a, C-4a), 152.1 (C-6), 160.6 (2'-C=O), 164.0 (2"-C=O), 170.1, 170.3, 170.6, 170.7 (4 × C(O)-CH₃).
MS (DCI, NH₃): *m*/*z* (%) = 882 (100) [M+NH₄]⁺, 865 (22) [M+H]⁺.

| | | | |
|---|---|---|---|
| C₄₅H₄₁N₄O₁₂Cl (865.29). | Ber. | C: 62.46 | H: 4.78 |
| | Gef. | C: 62.14 | H: 5.14 |

### Analytische Daten für das zweite Diastereomer 6b:

| | | |
|---|---|---|
| HPLC (semipräparativ): | Säule | Kromasil 100 C18, 5 µm, 250 × 8 mm |
| | Eluens | 72% Methanol in H₂O; 2.0 ml/min |
| | Rt | 29.74 min |

¹H-NMR (300 MHz, Aceton-d₆): δ =1.87, 1.94, 1.98, 2.10 (4 × s, 12 H, 4 × C(O)CH₃), 3.50-3.56 (m, 2 H, 1-Hₐ, 3-Hₐ), 3.87 (dd, *J* = 18.1, 5.6 Hz, 1 H, 1-H_{b}), 3.97-4.03 (m, 3 H, 5*-H, 6*-H₂), 4.32 (d, *J*= 7.9 Hz, 1 H, 1*-H), 4.65 (dd, *J*= 10.6, 3.4 Hz, 1 H, 3*-H), 4.93 (dd, *J*= 13.2, 3.8 Hz, 1 H, 3-H_{b}), 5.10 (m_{c}, 1 H, 2-H), 5.27 (dd, *J*= 3.8, 1.2 Hz, 1 H, 4*-H), 5.32 (dd, *J*= 10.6, 7.9 Hz, 1 H, 2*-H), 6.73 (d, *J*= 1.9 Hz, 1 H, 3'-H), 6.83 (s, 1 H, 5-H), 7.05-7.10 (m, 1 H, 5"-H), 7.19-7.25 (m, 1 H, 6"-H), 7.30 (d, *J*= 1.9 Hz, 1 H, 3"-H), 7.50-7.67 (m, 6 H, 4"-H, 7"-H, 6'-H, 7'-H, 8-H, 9-H), 7.95-8.00 (m, 2 H, 7-H, 10-H), 8.28 (s,1 H, 4'-H), 9.37 (s, 1 H, 5'-NH), 10.74 (s_{br}, 1 H, Indol-NH), 10.84 (s_{br}, 1 H, Indol-NH).
¹³C-NMR (125 MHz, Aceton-d₆): δ = 20.47, 20.48, 20.58, 20.71 (4 × C(O)CH₃), 34.66 (C-1), 50.81 (C-3), 55.94 (C-2), 62.10 (C-6*), 67.87 (C-4*), 68.86 (C-2*), 71.38 (C-3*), 71.61 (C-5*), 101.6 (C-1*), 103.6 (C-3"), 108.5 (C-3'), 108.9 (C-5), 113.1, 113.3 (C-6', C-7'), 113.9 (C-4'), 114.9 (C-10b), 120.4, 120.9, 122.5, 122.6, 123.7, 124.7, 126.1, 128.4 (C-7, C-8, C-9, C-10, C-4", C-5", C-6", C-7"), 124.5 (C-6a), 128.1, 128.8 (C-3'a, C-3"a), 132.5, 133.0, 133,2, 133.6, 134.6, 136.4, 137.9 (C-10a, C-5', C-2', C-2", C-7'a, C-7"a, C-4a), 152.2 (C-6), 160.5 (2'-C=O), 164.4 (2"-C=O), 170.2, 170.3, 170.6 170.7 (4 × C(O)-CH₃).

### rac-{2-Chlor-4-[5'-((1H-indol-2"-carbonyl)-amino)-1H-indol-2'-carbonyl]-1,2,3,4-tetrahydrobenzo[f]chinolin-6-yl}-β-D-galactopyranosid (7):

Das acetylgeschützte Galactosid **6** (40.0 mg, 46.2 µmol) wurde in absolutem Methanol (1.5 ml) gelöst und bei 0 °C mit NaOMe (3.77 µl einer 5.4 M Lösung in MeOH, 20.3 µmol, 0.44 Äq.) versetzt. Nach Entfernen der Kühlung ließ man 30 min bei Raumtemperatur rühren und fällte das Produkt anschließend durch Zugabe von Wasser aus. Der Niederschlag wurde über eine Fritte P2 abfiltriert und mit Wasser gewaschen. Zur Entfernung von Wasserspuren wurde der Feststoff dreimal in Toluol suspendiert und das Lösungsmittel im Vakuum entfernt. Man erhielt 30.5 mg (95 %) entschütztes Galactosid **7** in Form eines leicht gelblichen Feststoffes.
R_{f}= 0.28 (EE / MeOH =10 : 1)
UV (CH₃CN): λₘₐₓ (lg ε) = 215 nm (3.575), 310 (3.431).
IR (KBr): 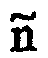 = 3406 cm⁻¹ (sehr breit, NH / OH), 3077 (Ar-H), 2924 (CH), 1622 (C=C), 1530, 14.04, 1233, 1074 748.
¹H-NMR (500 MHz, DMF-d₇): δ = 3.28 - 3.31 (m, 2 H, 3*-H, 5*-H), 3.47 (dd, *J=* 17.6, 4.8 Hz, 1 H, 1-Hₐ), 3.58 (dd, *J*= 11.0, 6.0 Hz, 1 H, 6*-Hₐ), 3.65 (dd, *J*=11.0, 6.4 Hz, 1 H, 6*-H_{b}), 3.82 - 3.88 (m, 2 H, 2*-H, 4*-H, 1-H_{b}), 4.43 (dd, *J*= 12.8, 6.4 Hz, 1 H, 3-Hₐ), 4.45 - 4.49 (m, 2 H, 1*-H, 3-H_{b}), 5.01 - 5.05 (m, 1 H, 2-H), 6.82 (d, *J=* 1.4 Hz, 1 H, 3'-H), 7.09 (ddd, *J*= 6.9, 6.9, 0.9 Hz, 1 H, 5"-H), 7.17 (s, 1 H, 5-H), 7.25 (ddd, *J*= 7.1,6.9,1.0 Hz, 1 H, 6"-H), 7.48 (m, 3 H, 3"-H, 7'-H, 8-H), 7.58 - 7.64 (m, 2 H, 7"-H, 9-H), 7.68 (d, *J*= 6.9 Hz, 1 H, 4"-H), 7.69 (d, *J=* 6.9, 1.9 Hz, 1 H, 6'-H), 7.97 (d, *J*= 8.5 Hz, 1 H, 10-H), 8.19 (d, *J*= 1.9 Hz, 1 H, 4'-H), 8.37 (d, *J*= 8.5 Hz, 1 H, 7-H), 10.20 (s, 1 H, 5'-NH), 11.67 ( s, 2 H, 2 × Indol-NH).
¹³C-NMR (125 MHz, DMF-d₇): δ = 39.33 (C-1), 51.87 (C-3), 55.54 (C-2), 61.53 (C-6*), 69.30, 71.69 (C-4*, C-2*), 74.54 (C-3*), 76.22 (C-5*), 103.9 (C-1*, C-3"), 107.4 (C-3'), 108.9 (C-5), 112.9, 113.0 (C-7', C-7"), 113.4 (C-4'), 114.4 (C-10b), 119.6 (C-6'), 120.5 (C-5"), 122.3 (C-4"), 123.2 (C-7), 123.5 (C-10), 124.2 (C-6"), 124.8 (C-6a), 125.2 (C-8), 127.8 (C-9), 128.5, 128.8 (C-3'a, C-3"a), 129.6 (C-Ta), 132.6, 133.0, 133.1, 134.7, (C-10a, C-5', C-2', C-2"), 136.5,137.8 (C-7"a, C-4a), 152.4 (C-6), 160.5 (2'-C=O), 164.5 (2"-C=O).
MS (FAB): *m*/*z* (%) = 695 (100) [M-H]⁻.

| | | | |
|---|---|---|---|
| C₃₇H₃₃N₄O₈Cl (697.14). | Ber. | C: 63.75 | H: 4.77 |
| | Gef. | C: 63.71 | H: 4.85 |

| | | |
|---|---|---|
| analytische HPLC zur Reinheitsbestimmung: | Säule | Kromasil 100 C18 |
| | Eluens | 62 % MeOH in H₂O; 0.7 ml/min |
| | RT | 41.65 min |

## Patentansprüche

1. 6-Hydroxy-2,3-dihydro-1H-indole der allgemeinen Formel (1), 5-Hydroxy-1,2-dihydro-3H-pyrrolo[3,2-e]indole der allgemeinen Formel (II) oder 5-Hydroxy-1,2-dihydro-3H-benzo[e]indole der allgemeinen Formel (III) ,oder deren O-Glykoside mit Mono-, Dioder Oligosacchariden der allgemeinen Formeln (IV), (V) und (VI), oder O-Glykoside mit Mono-, Di- oder Oligosacchariden von 6-Hydroxy-1,2,3,4-tetrahydro-benzo[*f*]-chinoline der allgemeinen Formeln (VII), oder 6-Hydroxy-1,2,3,4-tetrahydro-benzo[*f*]-chinoline der allgemeinen Formel (VIII): 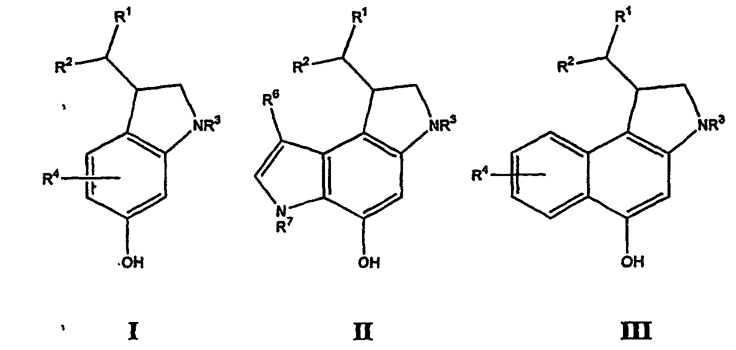 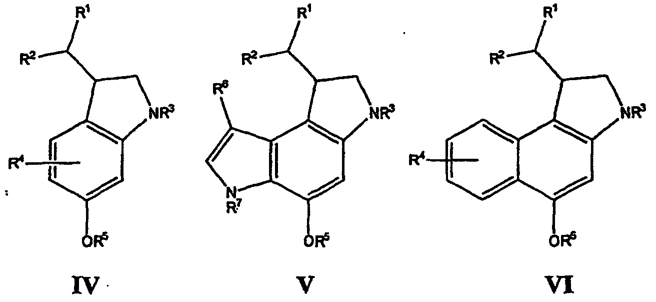 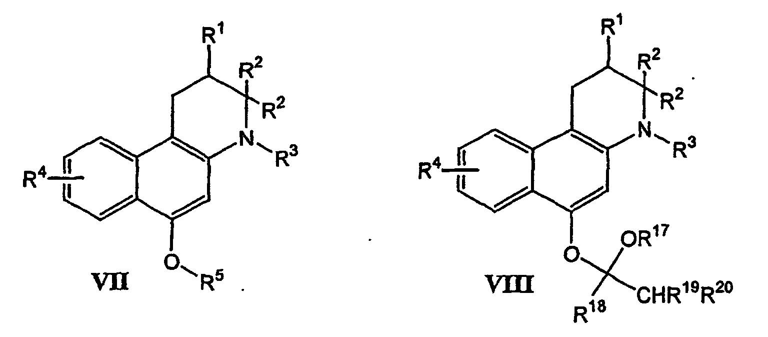 , wobei in den allgemeinen Formeln (I), (II), (III), (IV), (V), (VI), (VII) und (VIII)
R¹ für Halogen oder für eine Gruppe der Formel -OSO₂R⁸ steht,
wobei
R⁸ geradkettiges oder verzweigtes Alkyl mit bis zu 6 Kohlenstoffatomen, Benzyl oder Phenyl bedeutet, wobei letztere gegebenenfalls durch geradkettiges oder verzweigtes Alkyl mit bis zu 6 Kohlenstoffatomen substituiert sind,
R² für geradkettiges oder verzweigtes Alkyl mit bis zu 8 Kohlenstoffatomen steht, das gegebenenfalls durch Phenyl, Hydroxy, geradkettiges oder verzweigtes Alkoxy mit bis zu 6 Kohlenstoffatomen substituiert ist,
R³ für geradkettiges oder verzweigtes Alkyl mit bis zu 8 Kohlenstoffatomen steht, das gegebenenfalls durch Phenyl, Hydroxy, geradkettiges oder verzweigtes Alkoxy mit bis zu 6 Kohlenstoffatomen substituiert ist, oder für eine Gruppe der Formel -SO₂R⁸, -CO- R⁹ oder -CO₂R¹⁰ steht,
wobei
R⁸ die oben angegebene Bedeutung hat
und
R⁹ geradkettiges oder verzweigtes Alkyl mit bis zu 8 Kohlenstoffatomen bedeutet, das gegebenenfalls durch Carboxy, geradkettiges oder verzweigtes Alkoxycarbonyl mit bis zu 6 Kohlenstoffatomen oder durch eine Gruppe der Formel -CO-NR¹¹R¹² substituiert ist,
wobei den Rest die Formeln 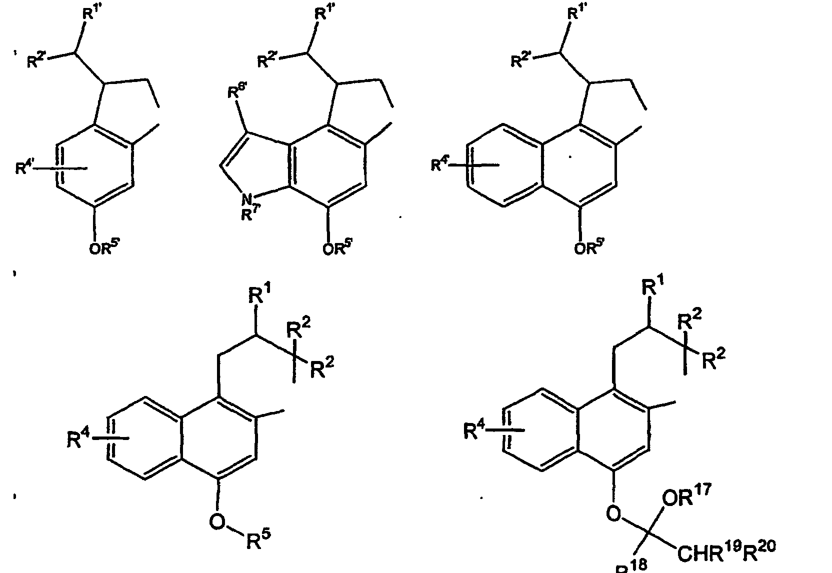 bilden,
wobei
R^{1'}, R^{2'}, R^{3'}, R^{4'}, R^{5'}, R^{6'} und R^{7'} die oben und im folgenden angegebenen Bedeutungen von R¹, R², R³, R⁴, R⁵ ,R⁶ und R⁷ haben, wobei R^{2'} in Erweiterung des oben Ausgeführten auch für ein Wasserstoff steht, und mit diesen gleich oder verschieden sind,
R¹⁰ Wasserstoff oder geradkettiges oder verzweigtes Alkyl mit bis zu 6 Kohlenstoffatomen bedeutet,
oder
R³ für einen Rest der Formeln 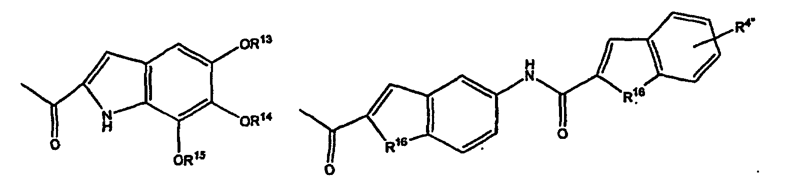 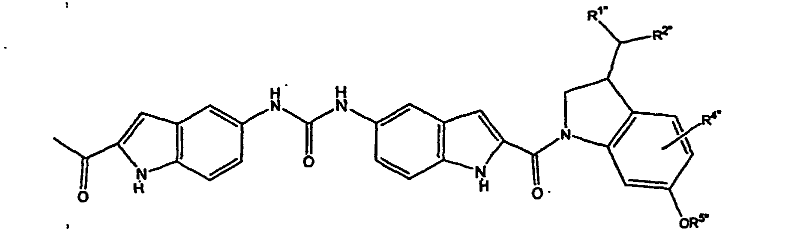 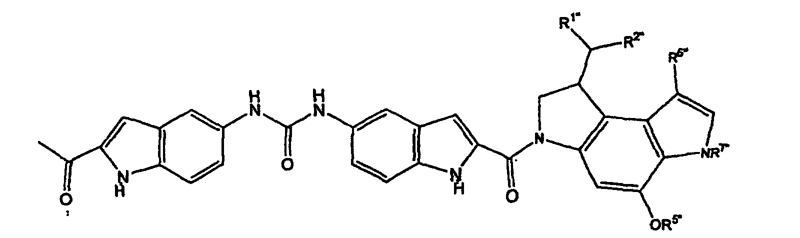 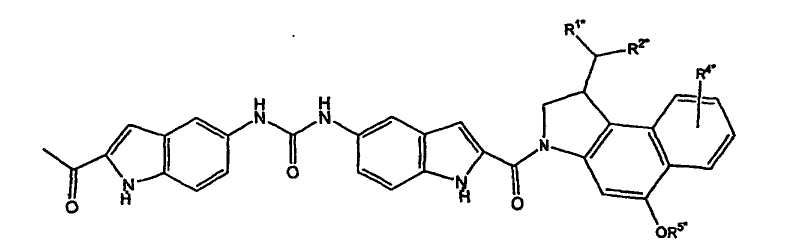 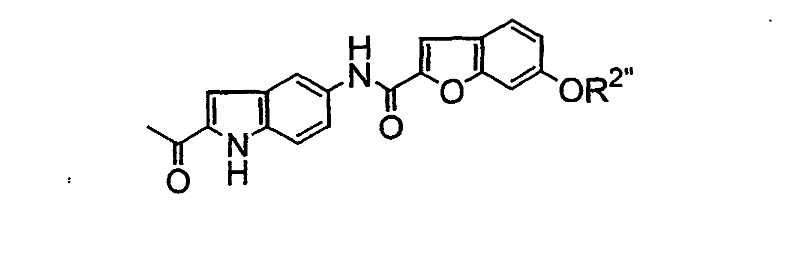 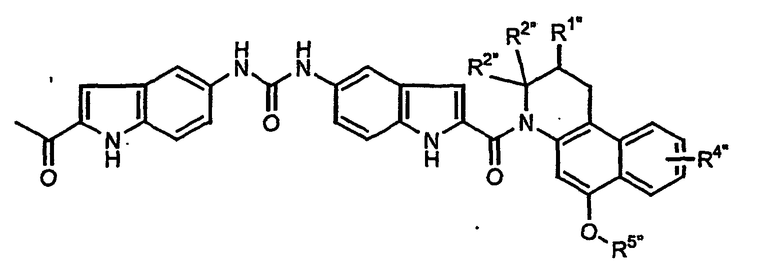 steht,
wobei
R^{1"}, R^{2"}, R^{4"}, R^{5"}, R^{6"} und R^{7"} die oben und im folgenden angegebenen Bedeutungen von R¹, R², R⁴, R⁵, R⁶ und R⁷ haben und mit diesen gleich oder verschieden sind,
R¹³, R¹⁴ und R¹⁵ gleich oder verschieden sind und Wasserstoff oder geradkettiges oder verzweigtes Alkyl oder Acyl mit jeweils bis zu 6 Kohlenstoffaotmen bedeuten,
R¹⁶ für unsubstituierten Stickstoff, Sauerstoff oder Schwefel steht,
R⁴ für Wasserstoff oder für geradkettiges oder verzweigtes Alkyl mit bis zu 8 Kohlenstoffatomen, das gegebenenfalls durch Hydroxy, Carboxy, Phenyl oder durch geradkettiges oder verzweigtes Alkoxy, Acyl oder Alkoxycarbonyl mit jeweils bis zu 6 Kohlenstoffatomen substituiert ist, oder für eine Hydroxy- oder Aminogruppe, die gegebenenfalls durch geradkettiges oder verzweigtes Alkyl, Acyl oder Alkoxycarbonyl mit jeweils bis zu 6 Kohlenstoffatomen substituiert ist, steht,
R⁵ für ein Monosaccharid oder Disaccharid oder Oligosaccharid von Hexosen oder Pentosen oder Heptosen steht, die auch zu der Gruppe der Desoxyzucker oder Aminozucker zählen können und zur D- oder L-Reihe gehören und in den Disacchariden oder Oligosacchariden entweder gleich oder verschieden sind,
R⁶ und R⁷ gleich oder verschieden sind und für Wasserstoff oder für geradkettiges Alkyl mit bis zu 8 Kohlenstoffatomen stehen.
R¹⁷ für eine Hydroxyschutzgruppe, oder für geradkettiges oder verzweigtes Alkyl mit bis zu 8 Kohlenstoffatomen steht, das gegebenenfalls durch Hydroxy, Carboxyl, Phenyl oder durch geradkettiges oder verzweigtes Alkoxyl, Acyl oder Alkoxycarbonyl mit jeweils bis zu 6 Kohlenstoffatomen substituiert ist, und gegebenenfalls mit R⁴, R⁵ oder R⁶ einen Ring bildet oder für einen Zuckerrest der Formel 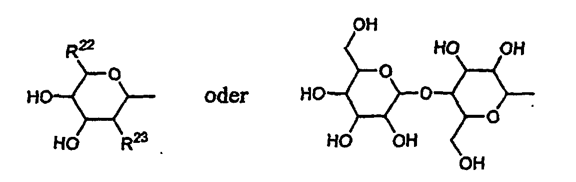 in der D- oder L-Form steht,
wobei
R²¹ Methyl oder die -CH₂OH-Gruppe bedeutet und
R²² Hydroxyl oder einen Rest der Formel -NR²³R²⁴ bedeutet, wobei
R²³ und R²⁴ gleich oder verschieden sind und Wasserstoff, geradkettiges oder verzweigtes Alkyl mit bis zu 6 Kohlenstoffatomen oder eine Aminoschutzgruppe bedeuten, und wobei die Hydroxygruppen der Zuckerreste gegebenen-falls geschützt sind,
R¹⁸, R¹⁹ und R²⁰ gleich oder verschieden sind und für Wasserstoff oder-geradkettiges oder verzweigtes oder cyclisches Alkyl mit bis zu 8 Kohlenstoffatomen stehen, das gegebenenfalls durch Phenyl, Halogen, Azido, geradkettiges oder verzweigtes Alkoxyl, Alkoxycarbonyl oder Oxyacyl mit jeweils bis zu 6 Kohlenstoffatomen, Hydroxyl, Carboxyl oder durch eine Gruppe der Formel -NR²³R²⁴ substituiert ist, wobei
R²³ und R²⁴ die oben angegebene Bedeutung haben.

2. Verbindung der allgemeinen Formel (I), (II), (III), (IV), (V), (VI), (VII) oder (VIII) nach Anspruch 1, wobei
R¹ für Chlor, Brom oder Jod steht,
R² für geradkettiges oder verzweigtes Alkyl mit bis zu 8 Kohlenstoffatomen steht, das gegebenenfalls durch Phenyl, Hydroxy, geradkettiges oder verzweigtes Alkoxy mit bis zu 4 Kohlenstoffatomen substituiert ist,
R³ für geradkettiges oder verzweigtes Alkyl mit bis zu 6 Kohlenstoffatomen steht, das gegebenenfalls durch Phenyl, Hydroxy, geradkettiges oder verzweigtes Alkoxy mit bis zu 4 Kohlenstoffatomen substituiert ist, oder für eine Gruppe der Formel -SO₂R⁸, -CO- R⁹ oder -CO₂R¹⁰ steht,
wobei
R⁸ geradkettiges oder verzweigtes Alkyl mit bis zu 4 Kohlenstoffatomen, Benzyl oder Phenyl bedeutet, wobei letztere gegebenenfalls durch geradkettiges oder verzweigtes Alkyl mit bis zu 4 Kohlenstoffatomen substituiert sind,
und
R⁹ geradkettiges oder verzweigtes Alkyl mit bis zu 6 Kohlenstoffatomen bedeutet, das gegebenenfalls durch Carboxy, geradkettiges oder verzweigtes Alkoxycarbonyl mit bis zu 4 Kohlenstoffatomen oder durch eine Gruppe der Formel -CO-NR¹¹R¹² substituiert ist,
wobei
R¹¹ und R¹² gleich oder verschieden sind und Wasserstoff oder geradkettiges oder verzweigtes Alkyl mit bis zu 3 Kohlenstoffatomen bedeuten,
oder
R¹¹ und R¹² gemeinsam mit dem Stickstoffatom den Rest der Formel 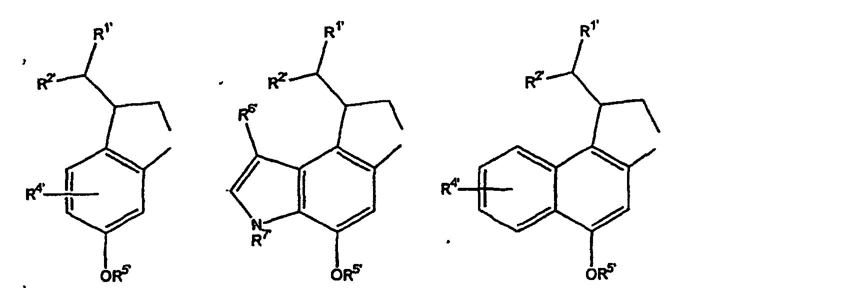 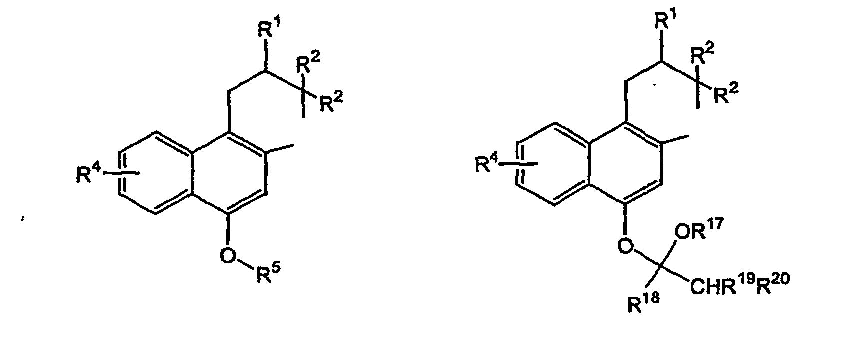 bilden,
wobei oder
R^{1'}, R^{2'}, R^{3'}, R^{4'}, R^{5'}, R^{6'} und R^{7'} die oben und im folgenden angegebenen Bedeutungen von R¹, R², R³, R4, R⁵ ,R⁶ und R⁷ haben, wobei R^{2'} in Erweiterung des oben Ausgeführten auch für ein Wasserstoff steht, und mit diesen gleich oder verschieden sind,
R¹⁰ Wasserstoff oder geradkettiges oder verzweigtes Alkyl mit bis zu 6 Kohlenstoffatomen bedeutet,
oder
R³ für einen Rest der Formel 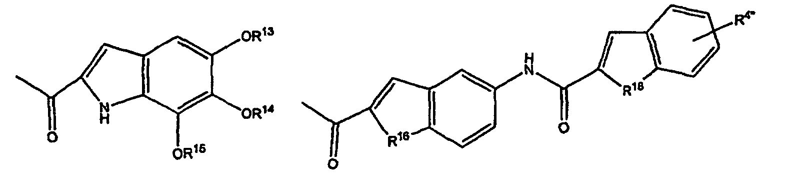 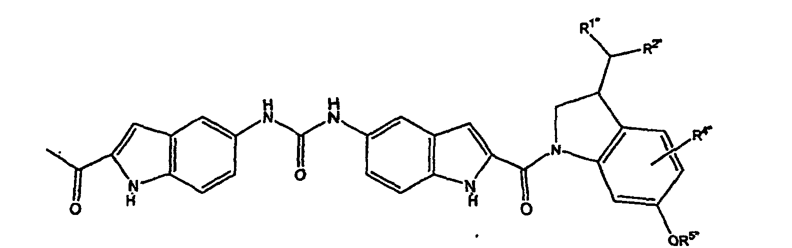 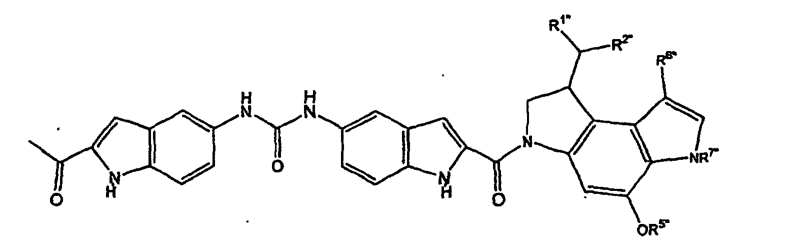 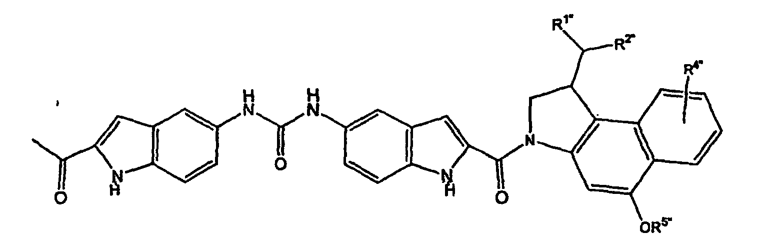 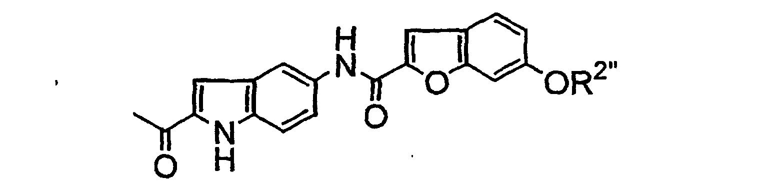 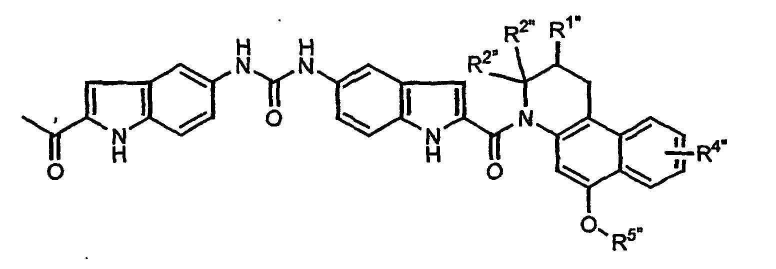 steht, wobei
R^{1"}, R^{2"}, R^{4"}, R^{5"}, R^{6"} und R^{7"} die oben und im folgenden angegebenen Bedeutungen von R¹, R^{2'}, R⁴, R⁵, R⁶ und R⁷ haben und mit diesen gleich oder verschieden sind,
R¹³, R¹⁴ und R¹⁵ gleich oder verschieden sind und Wasserstoff oder geradkettiges oder verzweigtes Alkyl oder Acyl mit jeweils bis zu 6 Kohlenstoffaotmen bedeuten,
R¹⁶ für unsubstituierten Stickstoff Sauerstoff oder Schwefel steht,
R⁴ für Wasserstoff oder geradkettiges oder verzweigtes Alkyl mit bis zu 6 Kohlenstoffatomen oder für eine Hydroxy- oder Aminogruppe steht, die gegebenenfalls durch ein geradkettiges oder verzweigtes Alkyl, Acyl oder Alkoxycarbonyl mit jeweils bis zu 6 Kohlenstoffatomen substituiert ist,
R⁵ für ein Monosaccharid oder ein Disaccharid von Hexosen oder Pentosen steht, die auch zu der Gruppe der Desoxy- oder Aminozucker zählen können und zur D- oder L-Reihe gehören und im Disaccharid gleich oder verschieden sein können,
R⁶ und R⁷ gleich oder verschieden sind und für Wasserstoff oder für geradkettiges Alkyl mit bis zu 8 Kohlenstoffatomen stehen.
R¹⁷ für eine Hydroxyschutzgruppe, oder für geradkettiges oder verzweigtes Alkyl mit bis zu 8 Kohlenstoffatomen steht, das gegebenenfalls durch Hydroxy, Carboxyl, Phenyl oder durch geradkettiges oder verzweigtes Alkoxyl, Acyl oder Alkoxycarbonyl mit jeweils bis zu 6 Kohlenstoffatomen substituiert ist, und gegebenenfalls mit R⁴, R⁵ oder R⁶ einen Ring bildet oder für einen Zuckerrest der Formel 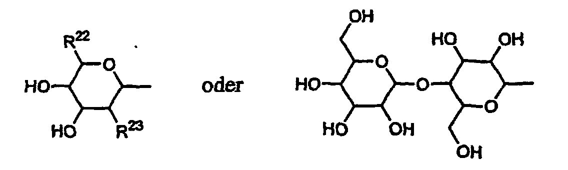 in der D- oder L-Form steht,
wobei
R²¹ Methyl oder die -CH₂OH-Gruppe bedeutet und
R²² Hydroxyl oder einen Rest der Formel -NR²³R²⁴ bedeutet, wobei
R²³ und R²⁴ gleich oder verschieden sind und Wasserstoff, geradkettiges oder verzweigtes Alkyl mit bis zu 6 Kohlenstoffatomen oder eine Aminoschutzgruppe bedeuten, und wobei die Hydroxygruppen der Zuckerreste gegebenen-falls geschützt sind,
R¹⁸, R¹⁹ und R²⁰ gleich oder verschieden sind und für Wasserstoff oder geradkettiges oder verzweigtes oder cyclisches Alkyl mit bis zu 8 Kohlenstoffatomen stehen, das gegebenenfalls durch Phenyl, Halogen, Azido, geradkettiges oder verzweigtes Alkoxyl, Alkoxycarbonyl oder Oxyacyl mit jeweils bis zu 6 Kohlenstoffatomen, Hydroxyl, Carboxyl oder durch eine Gruppe der Formel -NR²³R²⁴ substituiert ist, wobei
R²³ und R²⁴ die oben angegebene Bedeutung haben.

3. Verbindung der allgemeinen Formel (I), (II), (III), (IV), (V), (VI) oder (VII), nach Anspruch 1 oder 2, wobei
R¹ für Chlor steht,
R² für geradkettiges oder verzweigtes Alkyl mit bis zu 4 Kohlenstoffatomen steht, das gegebenenfalls durch Phenyl, Hydroxy, geradkettiges oder verzweigtes Alkoxy mit bis zu 3 Kohlenstoffatomen substituiert ist,
R³ für geradkettiges oder verzweigtes Alkyl mit bis zu 4 Kohlenstoffatomen steht, das gegebenenfalls durch Phenyl, Hydroxy, geradkettiges oder verzweigtes Alkoxy mit bis zu 3 Kohlenstoffatomen substituiert ist, oder für eine Grupppe der Formel -SO₂R⁸ steht,
wobei
R⁸ Methyl, Benzyl oder Phenyl bedeutet, wobei letztere gegebenenfalls durch Methyl oder Ethyl substituiert sind,
oder
R³ für einen Rest der Formel 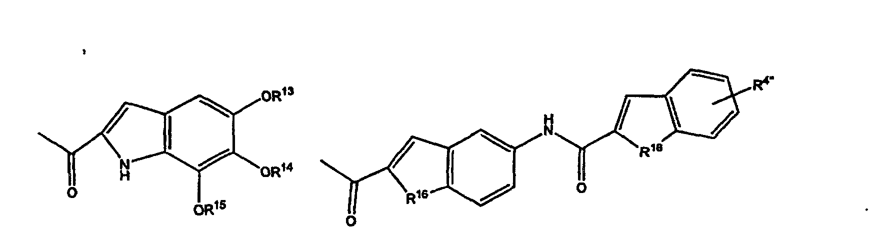 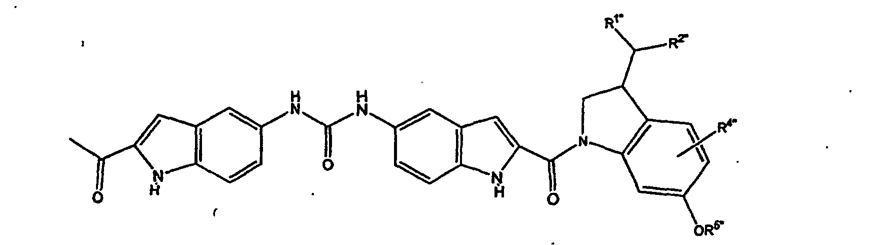 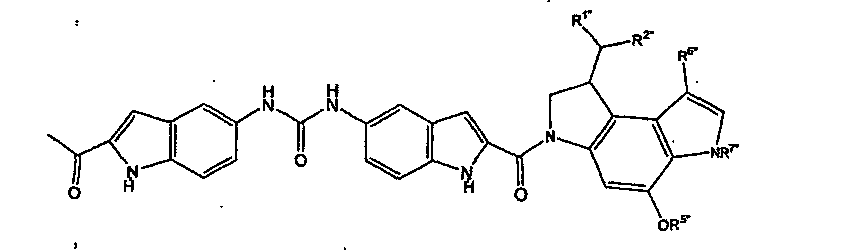 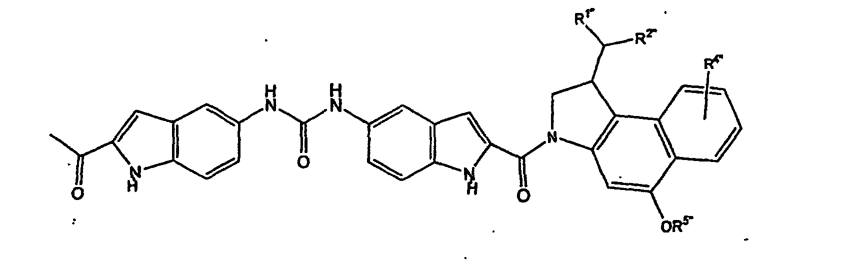 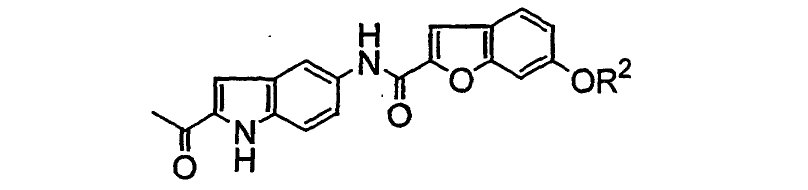 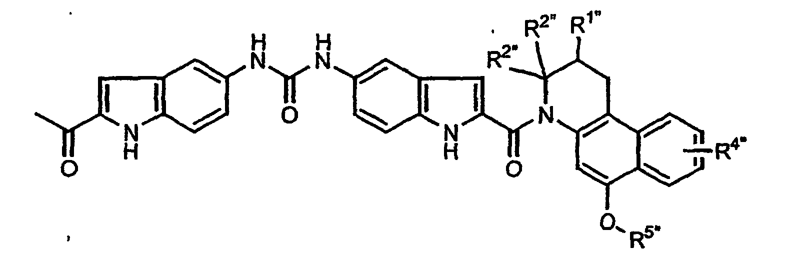 steht, wobei
R^{1"}, R^{2"}, R^{4"}, R^{5"}, R^{6"} und R^{7"} die oben und im folgenden angegebenen Bedeutungen von R¹, R^{2'}, R⁴, R⁵, R⁶ und R⁷ haben und mit diesen gleich oder verschieden sind,
R¹³, R¹⁴ und R¹⁵ gleich oder verschieden sind und Wasserstoff, Methyl, Ethyl oder Acetyl bedeuten,
R¹⁶ für unsubstituierten Stickstoff, Sauerstoff oder Schwefel steht,
R⁴ Wasserstoff bedeutet,
R⁵ für α-D-Mannose, β-D-Galaktose, β-D-Glucuronsäure und β-D-Glucose steht,
R⁶ und R⁷ gleich oder verschieden sind und für Wasserstoff oder ein geradkettiges oder verzweigtes Alkyl mit bis zu 4 Kohlenstoffatomen stehen.

4. Verfahren zur Herstellung einer Verbindung der allgemeinen Formel (I), (II), (III), (IV), (V) oder (VI) nach einem der Ansprüche 1 bis 3, **dadurch gekennzeichnet, dass** eine Verbindung der allgemeinen Formel (IX), (X) oder (XI) 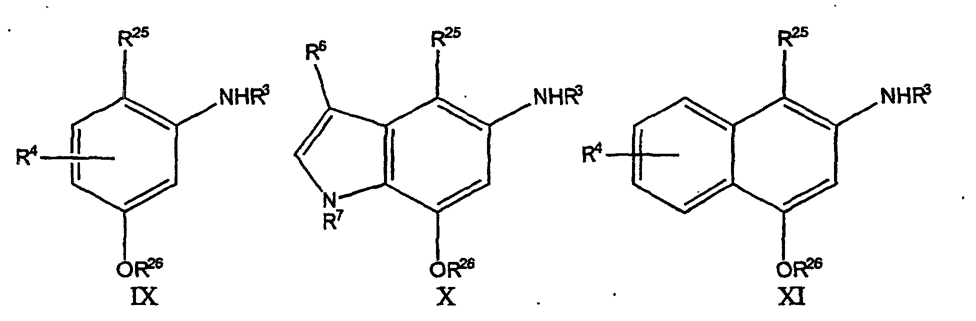 , wobei
R³ die oben angegebene Bedeutung hat, bevorzugt aber für *tert*.-Butoxycarbonyl steht,
R⁴, R⁶ und R⁷ die oben angegebene Bedeutung haben,
R²⁵ für Brom oder Iod steht,
R²⁶ für eine der oben angegebenen Hydroxyschutzgruppen, vorzugsweise aber für Benzyl, Benzyloxycarbonyl, 4-Nitrobenzyl oder 4-Methoxybenzyl steht,
nach ihrer Deprotonierung am Stickstoff mittels einer geeigneten Base mit einer Verbindung der allgemeinen Formel (XII) oder (XIII), 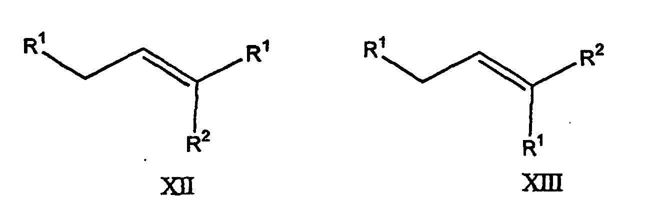 , wobei
R¹ die oben angegebene Bedeutung hat, bevorzugt aber für Chlor steht,
R² die oben angegebene Bedeutung hat,
in Anwesenheit eines organischen Lösemittels in eine Verbindung der allgemeinen Formel (XIV), (XV), (XVI), (XVII), (XVIII) oder (XIX) 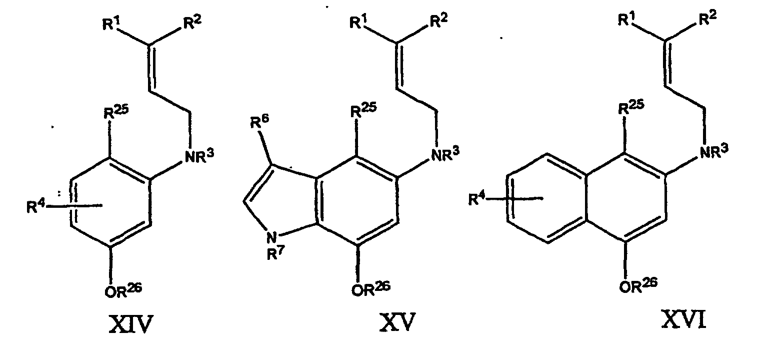 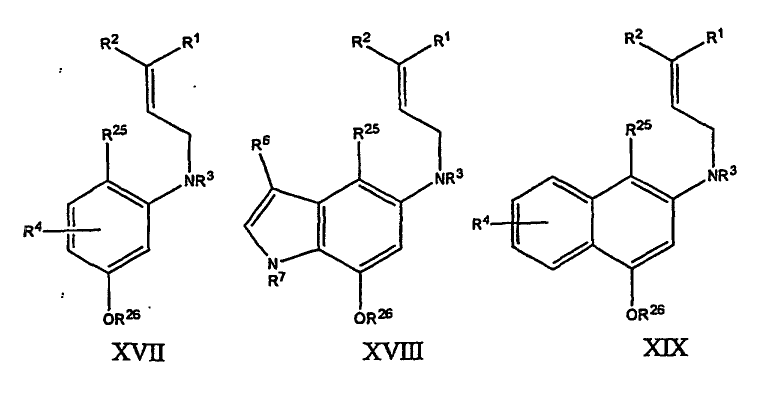 , wobei
R¹ die oben angegebene Bedeutung hat, bevorzugt aber für Chlor steht,
R² die oben angegebene Bedeutung hat,
R³ die oben angegebene Bedeutung hat, bevorzugt aber für *tert*.-Butoxycarbonyl steht,
R⁴, R⁶, R⁷, R²⁵und R²⁶ die oben angegebene Bedeutung haben,
überführt wird und diese anschließend durch eine radikalische Cyclisierung unter Einsatz von Tributylzinnhydrid und eines Radikalinitiators und schließlich durch Abspaltung der Schutzgruppen am phenolischen Sauerstoff durch Hydrogenolyse in Gegenwart von Pd/C und Ammoniumformiat in eine Verbindungen der allgemeinen Formel (I), (II) oder (III) überführt wird.

5. Verfahren nach Anspruch, **dadurch gekennzeichnet, dass** R¹ in der allgemeinen Formel (I), (II) oder (III) für Chlor steht

6. Verfahren nach Anspruch, **dadurch gekennzeichnet, dass** R² in der allgemeinen Formel (I), (II) oder (III) für *tert*.-Butoxycarbonyl steht,

7. Verfahren nach einem der Ansprüche 4 bis 6 zur Herstellung einer Verbindung der allgemeinen Formel (IV), (V) oder (VI), **dadurch gekennzeichnet, dass** die Verbindung der allgemeinen Formel (I), (II) oder (III) nachfolgend mit einem geschützten Mono-, Di- oder Oligosaccharid, das am C₁ eine Trichloracetimidatgruppe, ein Halogenatom oder eine anderer Abgangsgruppe trägt, umgesetzt wird und anschließend gegebenenfalls mit einer heteroaromatischen Carbonsäure in einem organischen Lösemittel in Anwesenheit eines Promotors und für einzelne Derivatisierungsschritte auch in Anwesenheit einer Base umgesetzt wird.

8. Verfahren zur Herstellung einer Verbindungen der allgemeinen Formel (VII) oder (VIII) nach Anspruch 1, **dadurch gekennzeichnet, daß** eineVerbindung der allgemeinen Formel (XX) 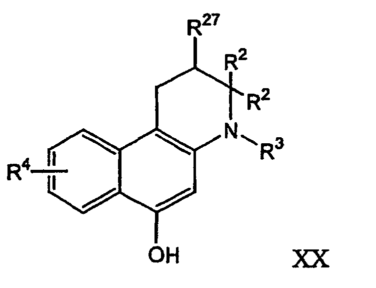 , wobei
R², R³ und R⁴ die oben angegebene Bedeutung haben, und
R²⁷ die oben angegebene Bedeutung von R¹ hat, vorzugsweise aber für Chlor oder OSi^{*t*}BuPh₂ oder OSiO^{*t*}BuPh₂ oder OSi^{*t*}BuMe₂ oder OCH₂Ph oder OCH₂-*p*-OMePh steht
im Fall, daß R¹⁸ in Struktur (VIII) für Wasserstoff steht, mit Enolethern der allgemeinen Formel (XXI) 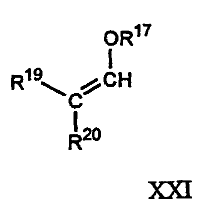 , wobei
R¹⁷, R¹⁹ und R²⁰ die oben angegebene Bedeutung haben
und
im Fall, daß R¹⁸ ≠ von Wasserstoff,
mit Enolethern der allgemeinen Formel (XXIa) 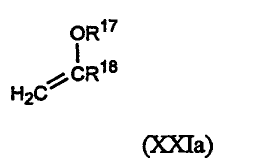 , wobei
R¹⁷ und R¹⁸ die oben angegebene Bedeutung haben, oder mit einem geschützten Mono-, Dioder Oligosaccharid, das an C₁ eine Trichloracetimidatgruppre oder ein Halogenatom oder eine andere Abgangsgruppe trägt, umgesetzt wird und anschließend gegebenenfalls mit einer heteroaromatischen Carbonsäure in einem organischen Lösemittel in Anwesenheit eines Katalysators und für einzelne Derivatisierungsschritte auch in Anwesenheit einer Base umgesetzt wird.

9. Mittel für die Krebstherapie enthaltend eine oder mehrere Verbindungen nach einem der Ansprüche 1 bis 3.

10. Verwendung einer oder mehrerer Verbindungen nach einem der Ansprüche 1 bis 3 zur Herstellung von Mitteln für die Krebstherapie.

## Claims

1. 6-Hydroxy-2,3-dihydro-1H-indoles of the general formula (I), 5-hydroxy-1,2-dihydro-3H-pyrrolo-[3,2-e]indoles of the general formula (II) or 5-hydroxy-1,2-dihydro-3H-benzo[e]indoles of the general formula (III), or the O-glycosides thereof with mono-, di- or oligosaccharides of the general formulae (IV), (V) and (VI), or O-glycosides with mono-, di- or oligosaccharides of 6-hydroxy-1,2,3,4-tetrahydrobenzo-[*f*]quinolines of the general formulae (VII), or 6-hydroxy-1,2,3,4-tetrahydrobenzo[*f*]quinolines of the general formula (VIII): 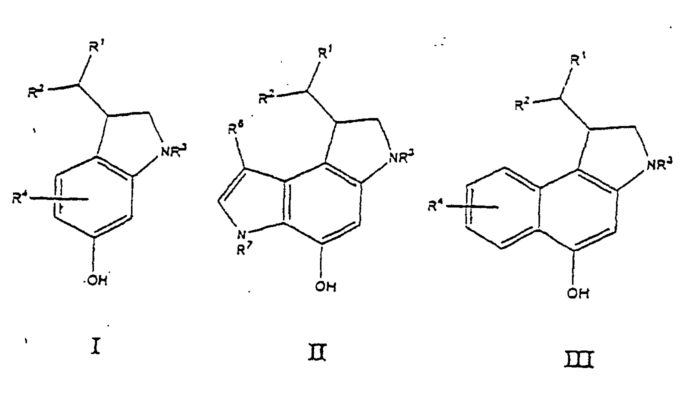 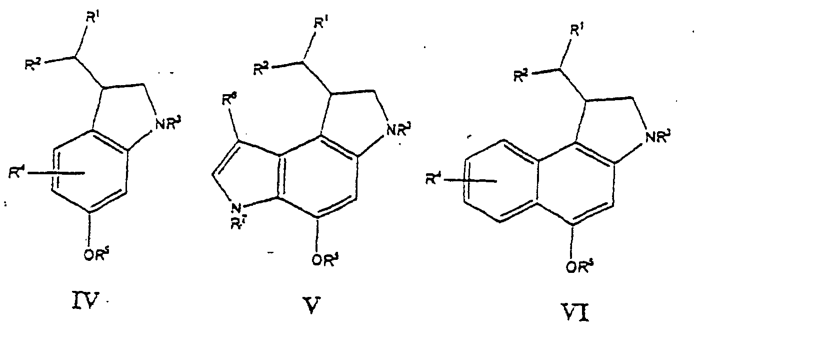 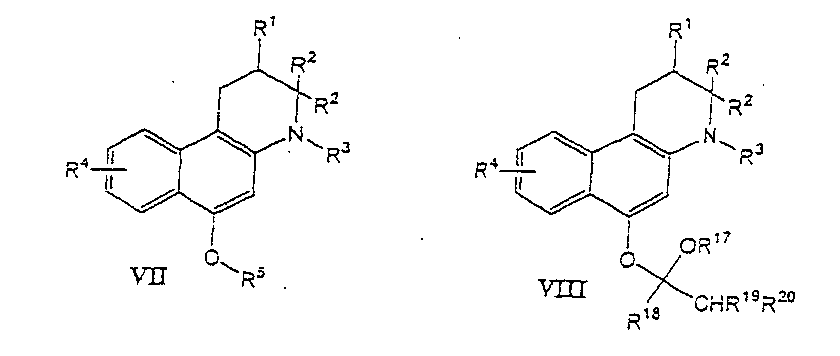 where, in the general formulae (I), (II), (III), (IV), (V), (VI), (VII) and (VIII),
R¹ is halogen or a group of the formula -OSO₂R⁸,
where
R⁸ is straight-chain or branched alkyl having up to 6 carbon atoms, benzyl or phenyl, each of the latter optionally being substituted by straight-chain or branched alkyl having up to 6 carbon atoms,
R² is straight-chain or branched alkyl having up to 8 carbon atoms, which is optionally substituted by phenyl, hydroxy, straight-chain or branched alkoxy having up to 6 carbon atoms,
R³ is straight-chain or branched alkyl having up to 8 carbon atoms, which is optionally substituted by phenyl, hydroxy, straight-chain or branched alkoxy having up to 6 carbon atoms, or is a group of the formula -SO₂R⁸, -CO-R⁹ or -CO₂R¹⁰,
where R⁸ has the meaning indicated above,
and
R⁹ is straight-chain or branched alkyl having up to 8 carbon atoms, which is optionally substituted by carboxy, straight-chain or branched alkoxycarbonyl having up to 6 carbon atoms or by a group of the formula -CO-NR¹¹R¹²,
where the formulae 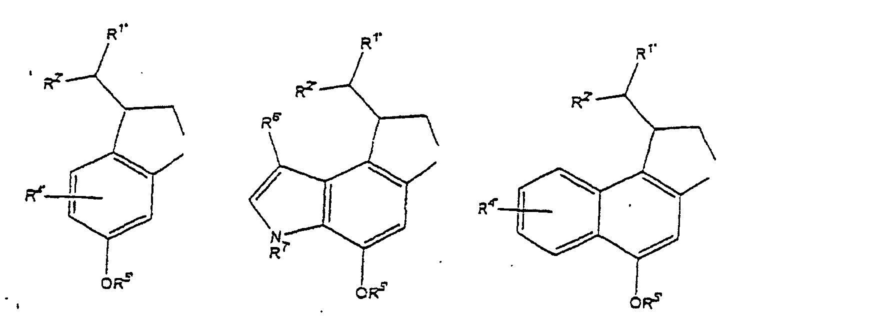 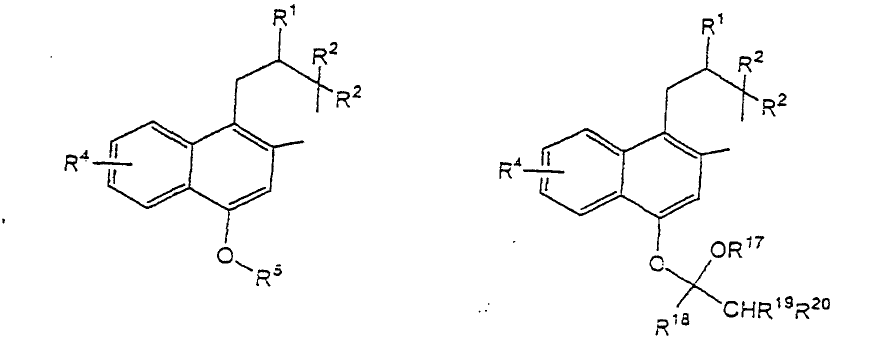 form the radical,
where
R^{1'}, R^{2'}, R^{3'}, R^{4'}, R^{5'}, R^{6'} and R^{7'} have the meanings indicated above and hereinafter of R¹, R², R³, R⁴, R⁵, R⁶ and R⁷, where R^{2'} in an extension of that stated above is also a hydrogen, and are identical to or different from the latter,
R¹⁰ is hydrogen or straight-chain or branched alkyl having up to 6 carbon atoms,
or
R³ is a radical of the formulae 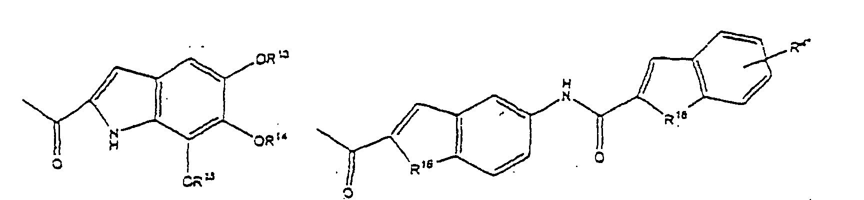 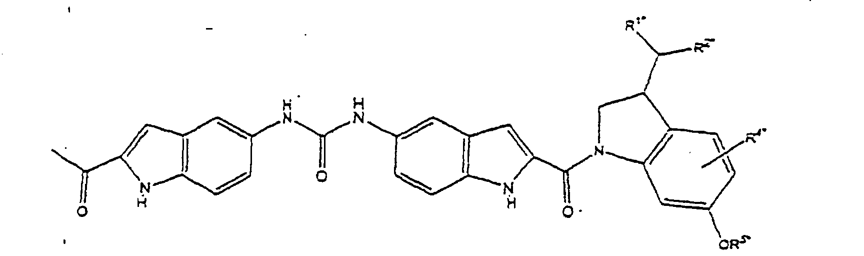 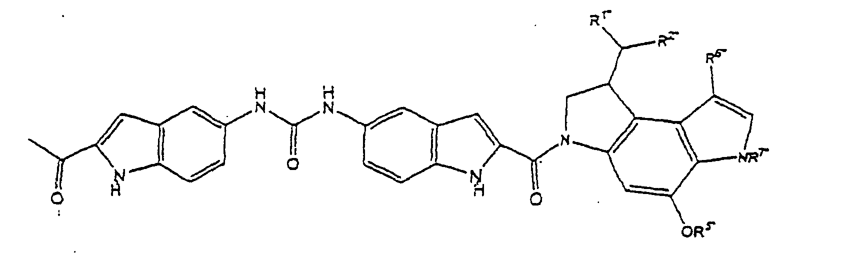 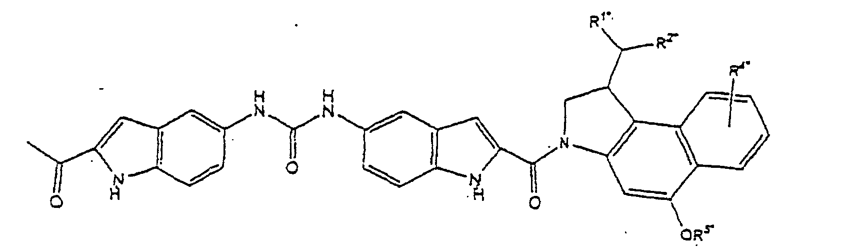 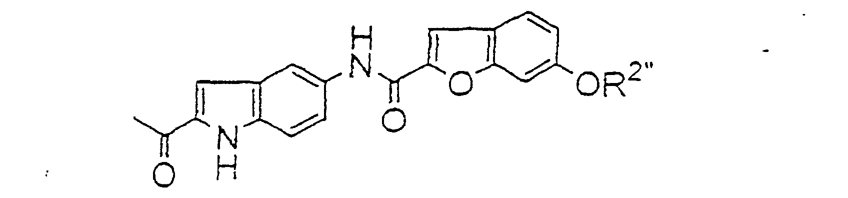 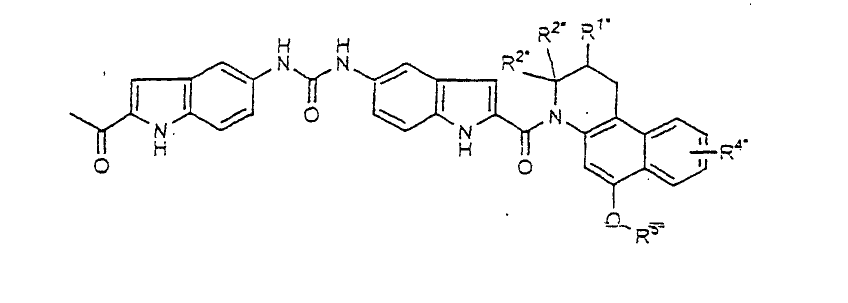 where
R^{1"}, R^{2"}, R^{4"}, R^{5"}, R^{6"} and R^{7"} have the meanings indicated above and hereinafter of R¹, R², R⁴, R⁵, R⁶ and R⁷ and are identical to or different from the latter,
R¹³, R¹⁴ and R¹⁵ are identical or different and are hydrogen or straight-chain or branched alkyl or acyl each having up to 6 carbon atoms,
R¹⁶ is unsubstituted nitrogen, oxygen or sulphur,
R⁴ is hydrogen or a straight-chain or branched alkyl having up to 8 carbon atoms, which is optionally substituted by hydroxy, carboxy, phenyl or by straight-chain or branched alkoxy, acyl or alkoxycarbonyl, each having up to 6 carbon atoms, or is a hydroxy or amino group which is optionally substituted by straight-chain or branched alkyl, acyl or alkoxycarbonyl each having up to 6 carbon atoms,
R⁵ is a monosaccharide or disaccharide or oligosaccharide of hexoses or pentoses or heptoses, which may also belong to the group of deoxy sugars or amino sugars, and belong to the D or L series, and, in the disaccharides or oligosaccharides, may be either identical or different,
R⁶ and R⁷ are identical or different and are hydrogen or are straight-chain alkyl having up to 8 carbon atoms,
R¹⁷ is a hydroxy protective group, or is straight-chain or branched alkyl having up to 8 carbon atoms, which is optionally substituted by hydroxy, carboxyl, phenyl or by straight-chain or branched alkoxyl, acyl or alkoxycarbonyl, each having up to 6 carbon atoms, and optionally forms with R⁴, R⁵ or R⁶ a ring, or is a saccharide residue of the formula 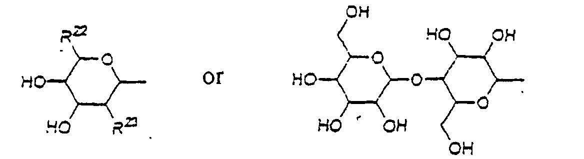 in the D or L form,
where
R²¹ is methyl or the -CH₂OH group, and
R²² is hydroxyl or a radical of the formula -NR²³R²⁴, where
R²³ and R²⁴ are identical or different and are hydrogen, straight-chain or branched alkyl having up to 6 carbon atoms or an amino protective group, and where the hydroxy groups of the saccharide residues are optionally protected,
R¹⁸, R¹⁹ and R²⁰ are identical or different and are hydrogen or straight-chain or branched or cyclic alkyl having up to 8 carbon atoms, which is optionally substituted by phenyl, halogen, azido, straight-chain or branched alkoxyl, alkoxycarbonyl or oxyacyl each having up to 6 carbon atoms, hydroxyl, carboxyl or by a group of the formula -NR²³R²⁴, where R²³ and R²⁴ have the meaning indicated above.

2. Compound of the general formula (I), (II), (III), (IV), (V) , (VI), (VII) or (VIII) according to Claim 1, where
R¹ is chlorine, bromine or iodine,
R² is straight-chain or branched alkyl having up to 8 carbon atoms, which is optionally substituted by phenyl, hydroxy, straight-chain or branched alkoxy having up to 4 carbon atoms,
R³ is straight-chain or branched alkyl having up to 6 carbon atoms, which is optionally substituted by phenyl, hydroxy, straight-chain or branched alkoxy having up to 4 carbon atoms, or is a group of the formula -SO₂R⁸, -CO-R⁹ or -CO₂R¹⁰,
where
R⁸ is straight-chain or branched alkyl having up to 4 carbon atoms, benzyl or phenyl, each of the latter optionally being substituted by straight-chain or branched alkyl having up to 4 carbon atoms,
and
R⁹ is straight-chain or branched alkyl having up to 6 carbon atoms, which is optionally substituted by carboxy, straight-chain or branched alkoxy-carbonyl having up to 4 carbon atoms or by a group of the formula -CO-NR¹¹R¹²,
where
R¹¹ and R¹² are identical or different and are hydrogen or straight-chain or branched alkyl having up to 3 carbon atoms,
or
R¹¹ and R¹² form together with the nitrogen atom the radical of the formula 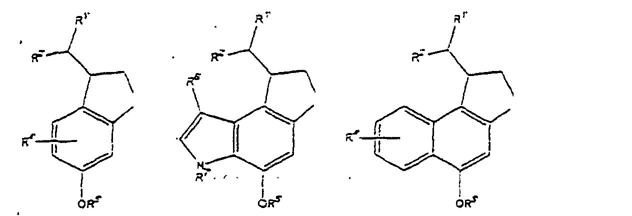 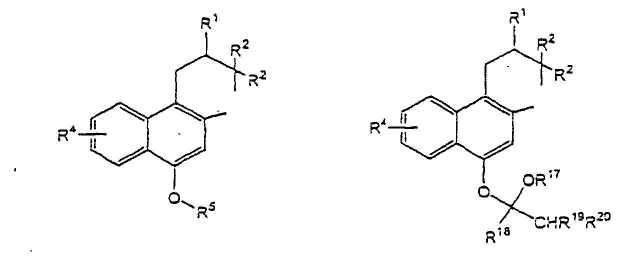 where
R^{1'}, R^{2'}, R^{3'}, R^{4'}, R^{5'}, R^{6'} and R^{7'} have the meanings indicated above and hereinafter of R¹, R², R³, R⁴, R⁵, R⁶ and R⁷, where R^{2'} in an extension of that stated above is also a -hydrogen, and are identical to or different from the latter,
R¹⁰ is hydrogen or straight-chain or branched alkyl having up to 6 carbon atoms,
or
R³ is a radical of the formula 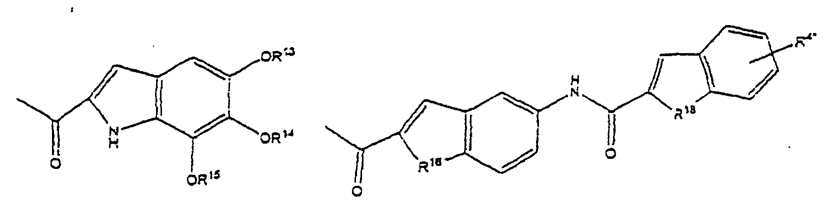 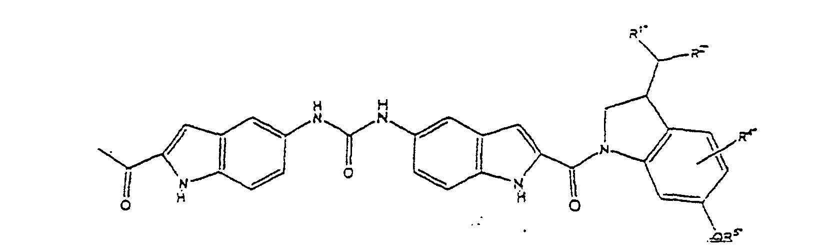 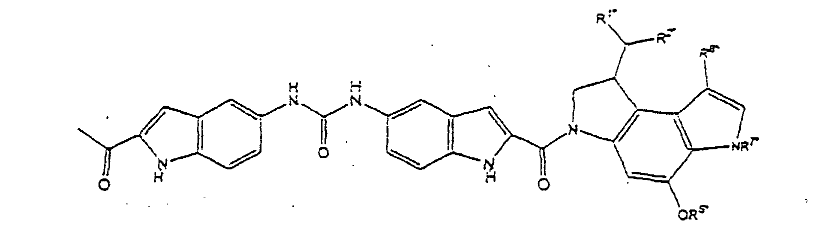 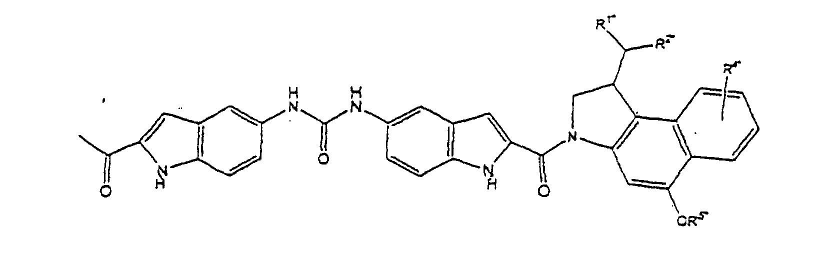 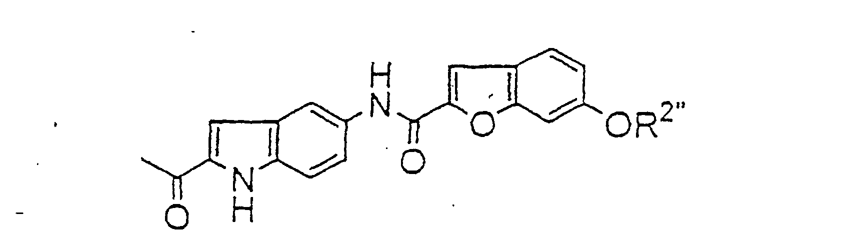 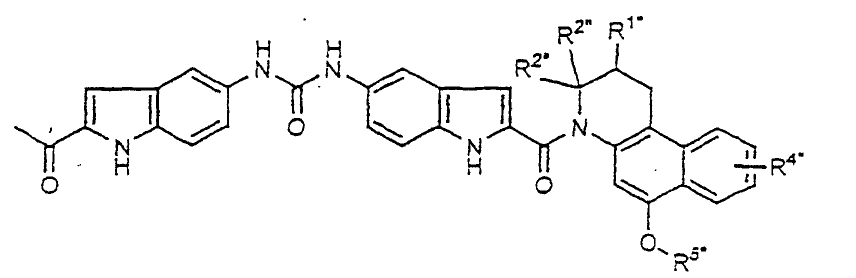
where
R^{1"}, R^{2"}, R^{4"}, R^{5"}, R^{6"} and R^{7"} have the meanings indicated above and hereinafter of R¹, R^{2'}, R⁴, R⁵, R⁶ and R⁷ and are identical to or different from the latter,
R¹³, R¹⁴ and R¹⁵ are identical or different and are hydrogen or straight-chain or branched alkyl or acyl each having up to 6 carbon atoms,
R¹⁶ is unsubstituted nitrogen, oxygen or sulphur,
R⁴ is hydrogen or straight-chain or branched alkyl having up to 6 carbon atoms or is a hydroxy or amino group which is optionally substituted by a straight-chain or branched alkyl, acyl or alkoxycarbonyl each having up to 6 carbon atoms,
R⁵ is a monosaccharide or a disaccharide of hexoses or pentoses, which may also belong to the group of deoxy or amino sugars, and belong to the D or L series, and in the disaccharide may be identical or different,
R⁶ and R⁷ are identical or different and are hydrogen or are straight-chain alkyl having up to 8 carbon atoms,
R¹⁷ is a hydroxy protective group, or is straight-chain or branched alkyl having up to 8 carbon atoms, which is optionally substituted by hydroxy, carboxyl, phenyl or by straight-chain or branched alkoxyl, acyl or alkoxycarbonyl, each having up to 6 carbon atoms, and optionally forms with R⁴, R⁵ or R⁶ a ring, or is a saccharide residue of the formula 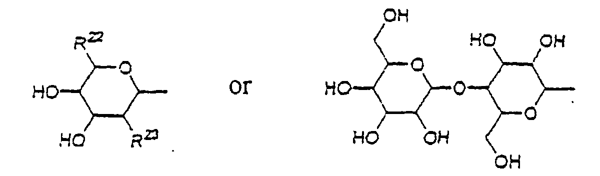 in the D or L form,
where
R²¹ is methyl or the -CH₂OH group, and
R²² is hydroxyl or a radical of the formula -NR²³R²⁴, where
R²³ and R²⁴ are identical or different and are hydrogen, straight-chain or branched alkyl having up to 6 carbon atoms or an amino protective group, and where the hydroxy groups of the saccharide residues are optionally protected,
R¹⁸, R¹⁹ and R²⁰ are identical or different and are hydrogen or straight-chain or branched or cyclic alkyl having up to 8 carbon atoms, which is optionally substituted by phenyl, halogen, azido, straight-chain or branched alkoxyl, alkoxycarbonyl or oxyacyl each having up to 6 carbon atoms, hydroxyl, carboxyl or by a group of the formula -NR²³R²⁴, where R²³ and R²⁴ have the meaning indicated above.

3. Compound of the general formula (I), (II), (III), (IV), (V), (VI) or (VII), according to Claim 1 or 2,
where
R¹ is chlorine,
R² is straight-chain or branched alkyl having up to 4 carbon atoms, which is optionally substituted by phenyl, hydroxy, straight-chain or branched alkoxy having up to 3 carbon atoms,
R³ is straight-chain or branched alkyl having up to 4 carbon atoms, which is optionally substituted by phenyl, hydroxy, straight-chain or branched alkoxy having up to 3 carbon atoms, or is a group of the formula -SO₂R⁸,
where
R⁸ is methyl, benzyl or phenyl, each of the latter optionally being substituted by methyl or ethyl,
or
R³ is a radical of the formula 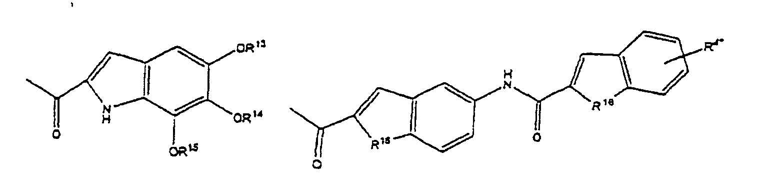 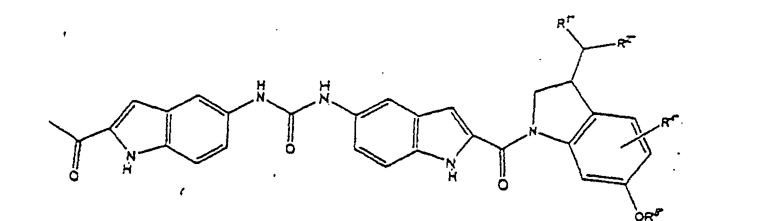 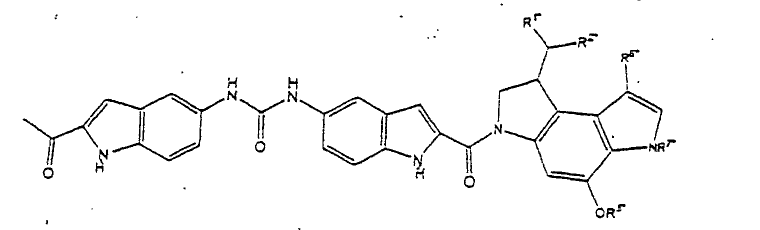 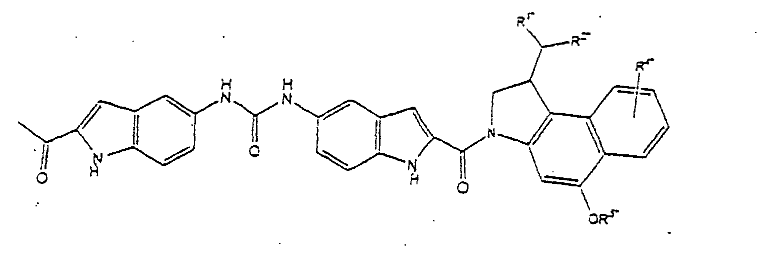 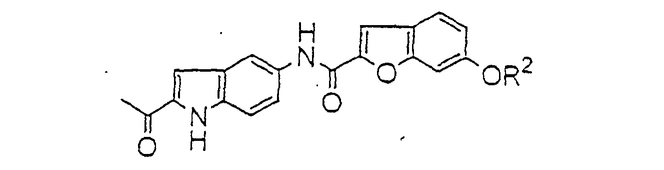 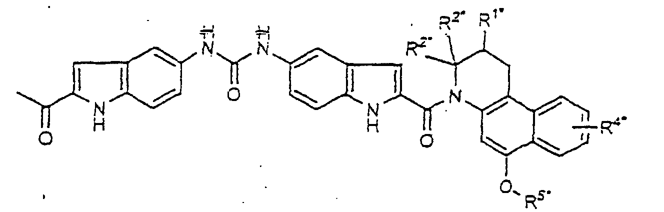 where
R^{1"}, R^{2"}, R^{4"}, R^{5"}, R^{6"} and R^{7"} have the meanings indicated above and hereinafter of R¹, R^{2'}, R⁴, R⁵, R⁶ and R⁷ and are identical to or different from the latter,
R¹³, R¹⁴ and R¹⁵ are identical or different and are hydrogen, methyl, ethyl or acetyl,
R¹⁶ is unsubstituted nitrogen, oxygen or sulphur,
R⁴ is hydrogen,
R⁵ is α-D-mannose, β-D-galactose, β-D-glucuronic acid and β-D-glucose,
R⁶ and R⁷ are identical or different and are hydrogen or a straight-chain or branched alkyl having up to 4 carbon atoms.

4. Process for preparing a compound of the general formula (I), (II), (III), (IV), (V) or (VI) according to any of Claims 1 to 3, **characterized in that** a compound of the general formula (IX), (X) or (XI) 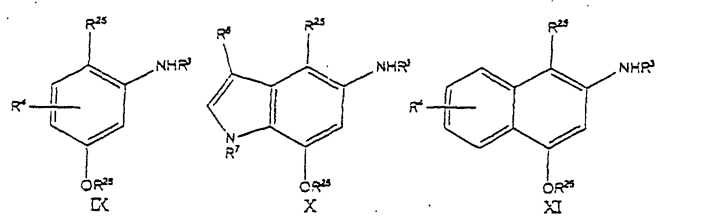 where
R³ has the meaning indicated above, but is preferably tert-butoxycarbonyl,
R⁴, R⁶ and R⁷ have the meaning indicated above,
R²⁵ is bromine or iodine,
R²⁶ is one of the hydroxy protective groups indicated above, but is preferably benzyl, benzyloxycarbonyl, 4-nitrobenzyl or 4-methoxybenzyl,
after deprotonation thereof on the nitrogen by means of a suitable base is converted with a compound of the general formula (XII) or (XIII) 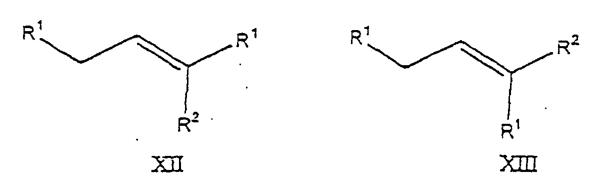 where
R¹ has the meaning indicated above, but is preferably chlorine,
R² has the meaning indicated above,
in the presence of an organic solvent into a compound of the general formula (XIV), (XV), (XVI), (XVII), (XVIII) or (XIX) 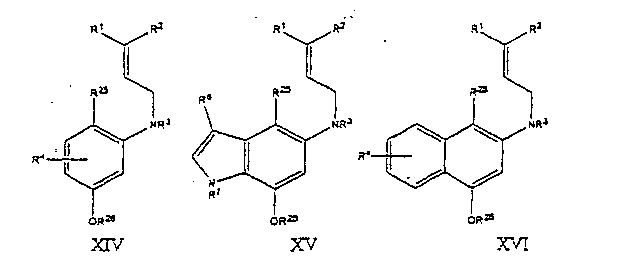 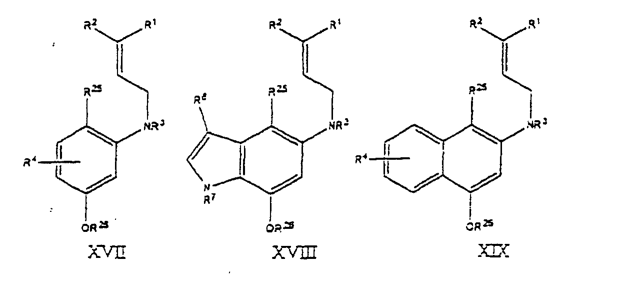 where
R¹ has the meaning indicated above, but is preferably chlorine,
R² has the meaning indicated above,
R³ has the meaning indicated above, but is preferably *tert*-butoxycarbonyl,
R⁴, R⁶, R⁷, R²⁵ and R²⁶ have the meaning indicated above, and the latter is subsequently converted by a free-radical cyclization using tributyltin hydride and a free-radical initiator and finally by eliminating the protective groups on the phenolic oxygen by hydrogenolysis in the presence of Pd/C and ammonium formate into a compounds of the general formula (I), (II) or (III).

5. Process according to Claim, **characterized in that** R¹ in the general formula (I), (II) or (III) is chlorine.

6. Process according to Claim, **characterized in that** R² in the general formula (I), (II) or (III) is tert-butoxycarbonyl.

7. Process according to any of Claims 4 to 6 for preparing a compound of the general formula (IV), (V) or (VI), **characterized in that** the compound of the general formula (I), (II) or (III) is subsequently reacted with a protected mono-, di- or oligosaccharide, which carries on C₁ a trichloroacetimidate group, a halogen atom or another leaving group, and is subsequently reacted where appropriate with a heteroaromatic carboxylic acid in an organic solvent in the presence of a promoter, and for some derivatization steps also in the presence of a base.

8. Process for preparing a compounds of the general formula (VII) or (VIII) according to Claim 1, **characterized in that** a compound of the general formula (XX) 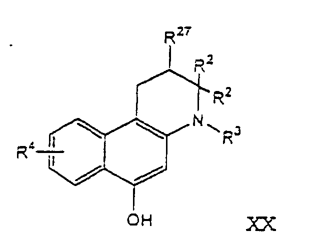 where
R², R³ and R⁴ have the meaning indicated above, and
R²⁷ has the meaning indicated above of R¹, but is preferably chlorine or OSi^{*t*}BuPh₂ or OSiO^{*t*}BuPh₂ or OSi^{*t*}BuMe₂ or OCH₂Ph or -OCH₂-*p*-OMePh,
in the case where R¹⁸ in structure (VIII) is hydrogen, is reacted with enol ethers of the general formula (XXI) 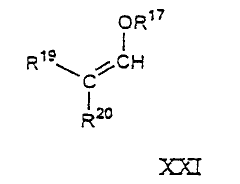 where
R¹⁷, R¹⁹ and R²⁰ have the meaning indicated above
and
in the case where R¹⁸ ≠ of hydrogen,
is reacted with enol ethers of the general formula (XXIa) 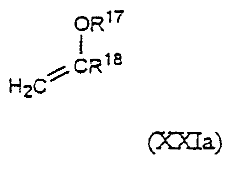 where
R¹⁷ and R¹⁸ have the meaning indicated above, or with a protected mono-, di- or oligosaccharide, which carries on C₁ a trichloroacetimidate group or a halogen atom or another leaving group, and is subsequently where appropriate reacted with a heteroaromatic carboxylic acid in an organic solvent in the presence of a catalyst, and.for some derivatization steps also in the presence of a base.

9. Composition for cancer therapy comprising one or more compounds according to any of Claims 1 to 3.

10. Use of one or more compounds according to any of Claims 1 to 3 for producing compositions for cancer therapy.

## Revendications

1. 6-hydroxy-2,3-dihydro-1H-indoles de formule générale (I), 5-hydroxy-1,2-dihydro-3H-pyrrolo[3,2-e]indoles de formule générale (II) ou 5-hydroxy-1,2-dihydro-3H-benzo[e]indoles de formule générale (III), ou leurs O-glycosides avec des mono-, di- ou oligosaccharides des formules générales (IV), (V) et (VI), ou O-glycosides avec des mono-, di- ou oligosaccharides de 6-hydroxy-1,2,3,4-tétrahydro-benzo[*f*]-quinoléines des formules générales (VII), ou 6-hydroxy-1,2,3,4-tétrahydro-benzo[F]-quinoléines de formule générale (VIII) : 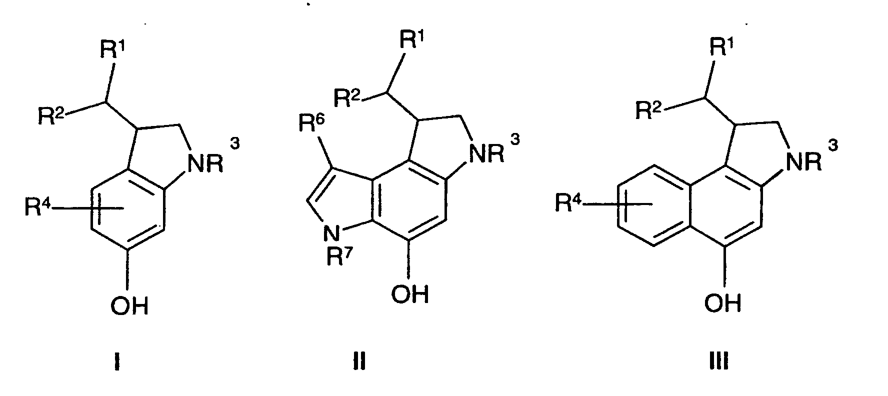 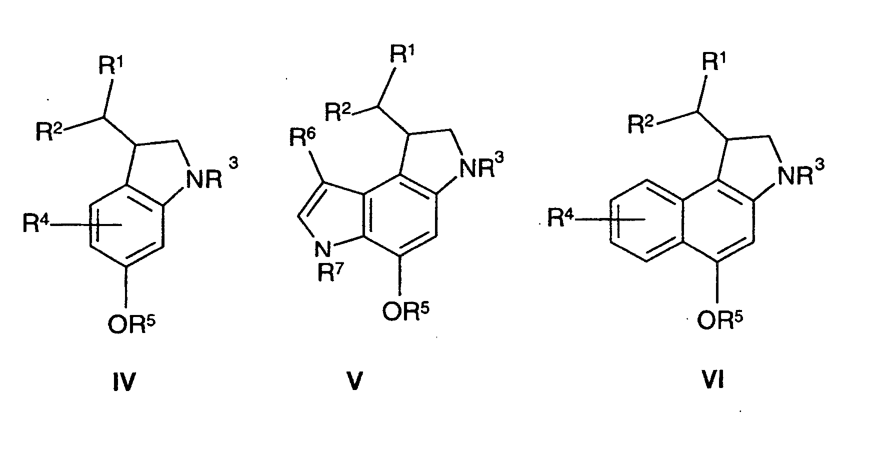 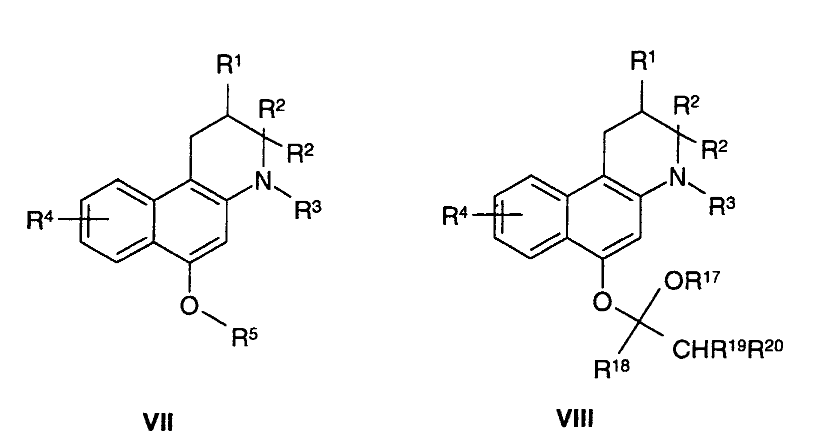 où, dans les formules générales (I), (II), (III), (IV), (V), (VI), (VII) et (VIII), R¹ représente halogène ou un groupe de formule -OSO₂R⁸,
où
R⁸ représente un alkyle linéaire ou ramifié ayant jusqu'à 6 atomes de carbone, benzyle ou phényle, où ces derniers sont éventuellement substitués par alkyle linéaire ou ramifié ayant jusqu'à 6 atomes de carbone,
R² représente alkyle linéaire ou ramifié ayant jusqu'à 8 atomes de carbone, qui est éventuellement substitué par phényle, hydroxyle, alcoxy linéaire ou ramifié ayant jusqu'à 6 atomes de carbone,
R³ représente alkyle linéaire ou ramifié ayant jusqu'à 8 atomes de carbone, qui est éventuellement substitué par phényle, hydroxyle, alcoxy linéaire ou ramifié ayant jusqu'à 6 atomes de carbone, ou représente un groupe de formule -SO₂R⁸, -CO-R⁹ ou -CO₂R¹⁰,
où
R⁸ a la signification indiquée ci-dessus
et
R⁹ représente alkyle linéaire ou ramifié ayant jusqu'à 8 atomes de carbone qui est éventuellement substitué par carboxyle, alcoxycarbonyle linéaire ou ramifié ayant jusqu'à 6 atomes de carbone ou par un groupe de formule -CO-NR¹¹R¹²,
où les formules 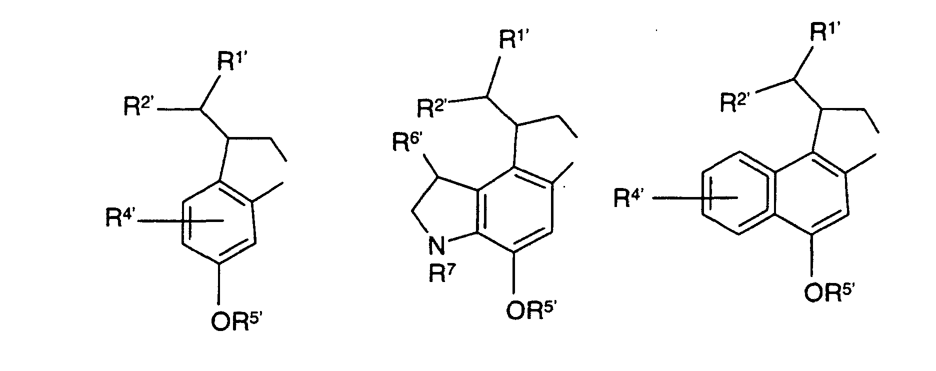 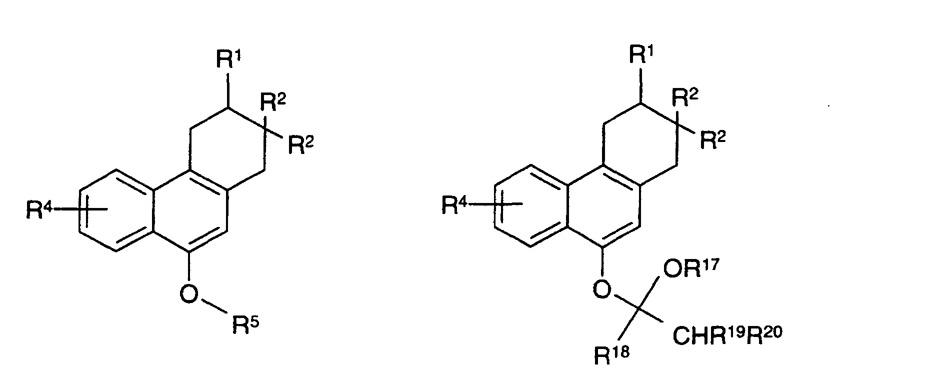 forment le reste,
où
R^{1'}, R^{2'}, R^{3'}, R^{4'}, R^{5'}, R^{6'} et R^{7'} ont les significations de R¹, R², R³, R⁴, R⁵, R⁶ et R⁷ indiquées ci-dessus et dans la suite, où R^{2'}, en plus de ce qui a été indiqué ci-dessus, représente aussi un hydrogène, et sont identiques ou différents de ceux-ci,
R¹⁰ représente l'hydrogène ou alkyle linéaire ou ramifié ayant jusqu'à 6 atomes de carbone,
ou bien
R³ représente un reste des formules 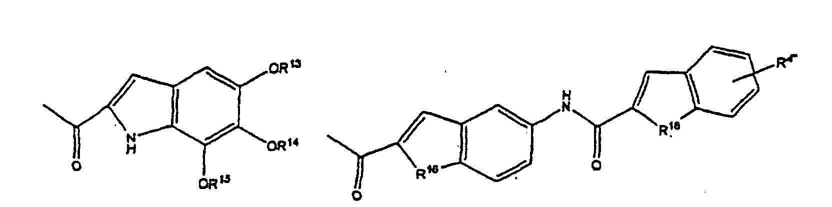 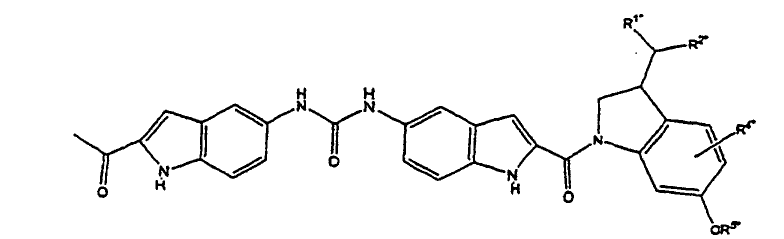 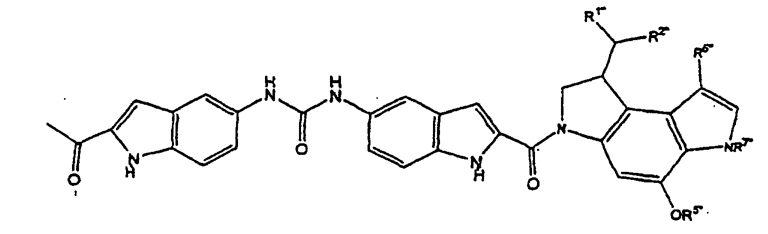 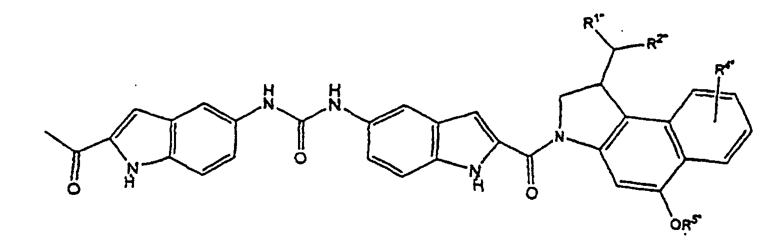 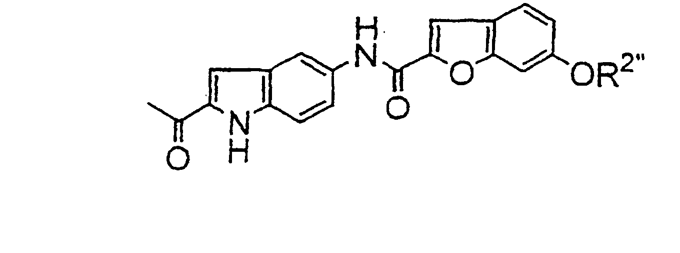 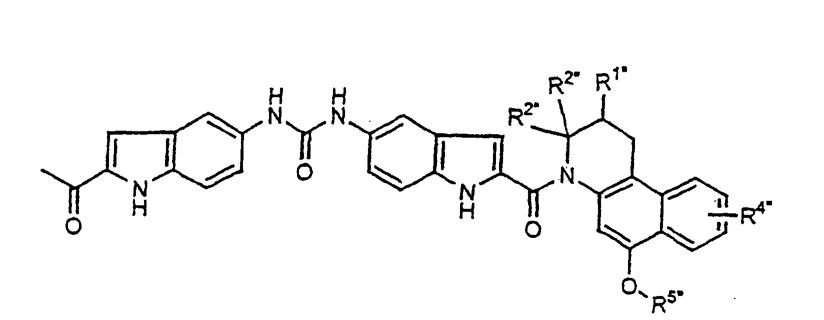 où
R^{1"}, R^{2"}, R^{4"}, R^{5"}, R^{6"} et R^{7"} ont les significations de R¹, R², R⁴, R⁵, R⁶ et R⁷ indiquées ci-dessus et dans la suite, et sont identiques ou différents de ceux-ci,
R¹³, R¹⁴ et R¹⁵ sont identiques ou différentes et représentent l'hydrogène ou alkyle ou acyle linéaire ou ramifié ayant dans chaque cas jusqu'à 6 atomes de carbone,
R¹⁶ représente l'azote, l'oxygène ou le soufre non substitué,
R⁴ représente l'hydrogène ou alkyle linéaire ou ramifié ayant jusqu'à 8 atomes de carbone, qui est éventuellement substitué par hydroxyle, carboxyle, phényle ou par alcoxy, acyle ou alcoxycarbonyle linéaire ou ramifié ayant dans chaque cas jusqu'à 6 atomes de carbone, ou un groupe hydroxyle ou amino qui est éventuellement substitué par alkyle, acyle ou alcoxycarbonyle linéaire ou ramifié ayant dans chaque cas jusqu'à 6 atomes de carbone,
R⁵ représente un monosaccharide ou un disaccharide ou un oligosaccharide d'hexoses ou de pentoses ou d'heptoses, qui peuvent aussi faire partie du groupe des désoxysucres ou des aminosucres et qui appartiennent à la série D ou L et qui sont identiques ou différents concernant les disaccharides ou les oligosaccharides,
R⁶ et R⁷ sont identiques ou différents et représentent l'hydrogène ou alkyle linéaire ayant jusqu'à 8 atomes de carbone,
R¹⁷ représente un groupe protecteur d'hydroxyle ou alkyle linéaire ou ramifié ayant jusqu'à 8 atomes de carbone, qui est éventuellement substitué par hydroxyle, carboxyle, phényle ou par alcoxy, acyle ou alcoxycarbonyle linéaire ou ramifié ayant dans chaque cas jusqu'à 6 atomes de carbone, et forme éventuellement avec R⁴, R⁵ ou R⁶ un cycle ou représente un reste de sucre de formule 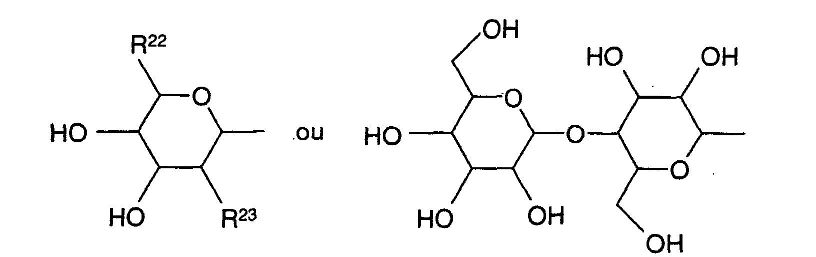 sous la forme D ou L,
où
R²¹ représente méthyle ou le groupe -CH₂OH et
R²² représente hydroxyle ou un reste de formule -NR²³R²⁴, où
R²³ et R²⁴ sont identiques ou différents et représentent l'hydrogène, alkyle linéaire ou ramifié ayant jusqu'à 6 atomes de carbone ou un groupe protecteur d'amino, et où les groupes hydroxyle des restes de sucre sont éventuellement protégés,
R¹⁸, R¹⁹ et R²⁰ sont identiques ou différents et représentent l'hydrogène ou alkyle linéaire ou ramifié ou cyclique ayant jusqu'à 8 atomes de carbone, qui est éventuellement substitué par phényle, halogène, azido, alcoxy, alcoxycarbonyle ou oxyacyle linéaire ou ramifié ayant dans chaque cas jusqu'à 6 atomes de carbone, hydroxyle, carboxyle ou par un groupe de formule -NR²³R²⁴, où R²³ et R²⁴ ont la signification indiquée ci-dessus.

2. Composé de formule générale (I), (II), (III), (IV), (V), (VI), (VII) ou (VIII) selon la revendication 1 où
R¹ représente le chlore, le brome ou l'iode,
R² représente alkyle linéaire ou ramifié ayant jusqu'à 8 atomes de carbone, qui est éventuellement substitué par phényle, hydroxyle, alcoxy linéaire ou ramifié ayant jusqu'à 4 atomes de carbone,
R³ représente alkyle linéaire ou ramifié ayant jusqu'à 6 atomes de carbone, qui est éventuellement substitué par phényle, hydroxyle, alcoxy linéaire ou ramifié ayant jusqu'à 4 atomes de carbone, ou représente un groupe de formule -SO₂R⁸, -CO-R⁹ ou -CO₂R¹⁰,
où
R⁸ représente alkyle linéaire ou ramifié ayant jusqu'à 4 atomes de carbone, benzyle ou phényle où ces derniers sont éventuellement substitués par alkyle linéaire ou ramifié ayant jusqu'à 4 atomes de carbone,
et
R⁹ représente alkyle linéaire ou ramifié ayant jusqu'à 6 atomes de carbone, qui est éventuellement substitué par carboxyle, alcoxycarbonyle linéaire ou ramifié ayant jusqu'à 4 atomes de carbone ou par un groupe de formule -CO-NR¹¹R¹²,
où
R¹¹ et R¹² sont identiques ou différents et représentent l'hydrogène ou alkyle linéaire ou ramifié ayant jusqu'à 3 atomes de carbone,
ou
R¹¹ et R¹² forment avec l'atome d'azote le reste de formule 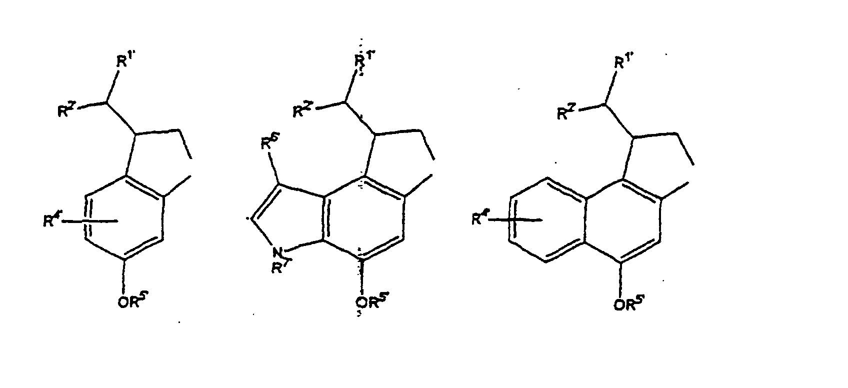 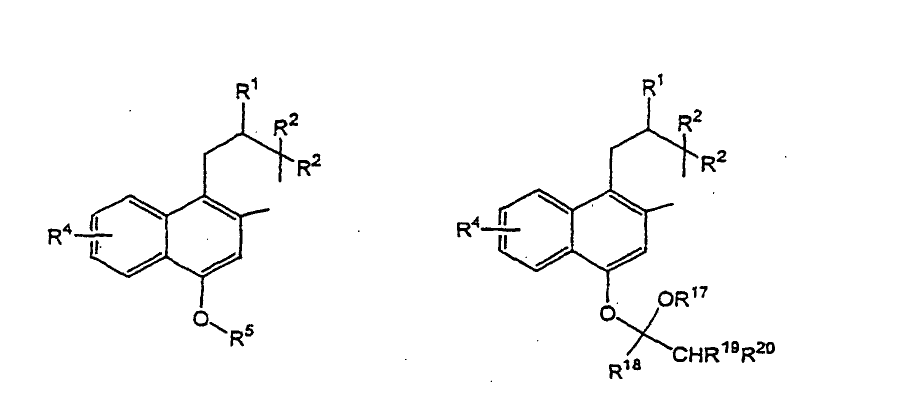 où
R^{1'}, R^{2'}, R^{3'}, R^{4'}, R^{5'}, R^{6'} et R^{7'} ont les significations de R¹, R², R³, R⁴, R⁵, R⁶ et R⁷ indiquées ci-dessus et dans la suite, où R^{2'}, en plus de ce qui a été indiqué ci-dessus, représente aussi un hydrogène, et sont identiques ou différents de ceux-ci,
R¹⁰ représente hydrogène ou alkyle linéaire ou ramifié ayant jusqu'à 6 atomes de carbone,
ou bien,
R³ représente un reste de formule 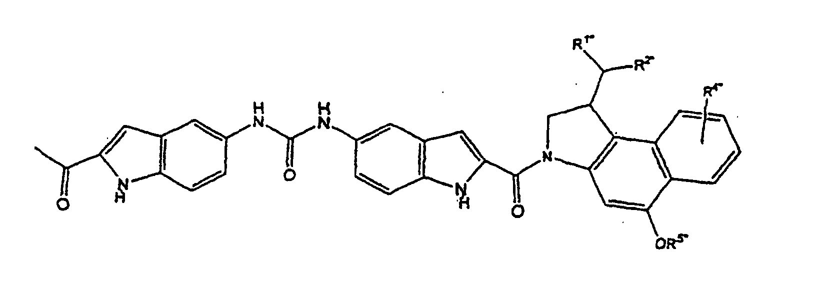 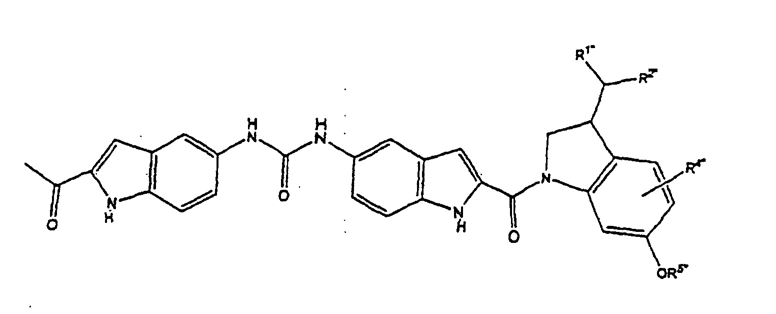 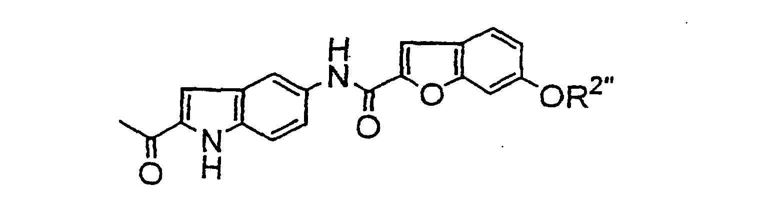 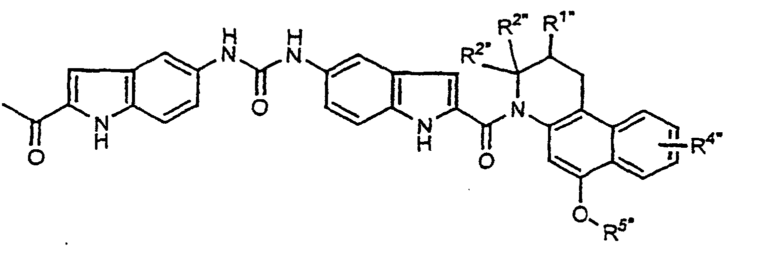 où
R^{1"}, R^{2"}, R^{4"}, R^{5"}, R^{6"}et R^{7"} ont les significations de R¹, R^{2'}, R⁴, R⁵, R⁶ et R⁷ indiquées ci-dessus et dans la suite et sont identiques ou différents de ceux-ci,
R¹³, R¹⁴ et R¹⁵ sont identiques ou différents et représentent hydrogène ou alkyle ou acyle linéaire ou ramifié ayant dans chaque cas jusqu'à 6 atomes de carbone,
R¹⁶ représente l'azote, l'oxygène ou le soufre non substitué,
R⁴ représente l'hydrogène ou alkyle linéaire ou ramifié ayant jusqu'à 6 atomes de carbone ou un groupe hydroxyle ou amino qui est éventuellement substitué par alkyle, acyle ou alcoxycarbonyle linéaire ou ramifié ayant dans chaque cas jusqu'à 6 atomes de carbone,
R⁵ représente un monosaccharide ou un disaccharide d'hexoses ou de pentoses, qui peuvent faire partie aussi du groupe des désoxy- ou aminosucres et appartiennent à la série D ou L et qui peuvent être identiques ou différents concernant le disaccharide,
R⁶ et R⁷ sont identiques ou différents et représentent l'hydrogène ou alkyle linéaire ayant jusqu'à 8 atomes de carbone,
R¹⁷ représente un groupe protecteur d'hydroxyle, ou alkyle linéaire ou ramifié ayant jusqu'à 8 atomes de carbone, qui est éventuellement substitué par hydroxyle, carboxyle, phényle ou par alcoxy acyle ou alcoxycarbonyle linéaire ou ramifié ayant dans chaque cas jusqu'à 6 atomes de carbone, et forme éventuellement un cycle avec R⁴, R⁵ ou R⁶ ou représente un reste de sucre de formule 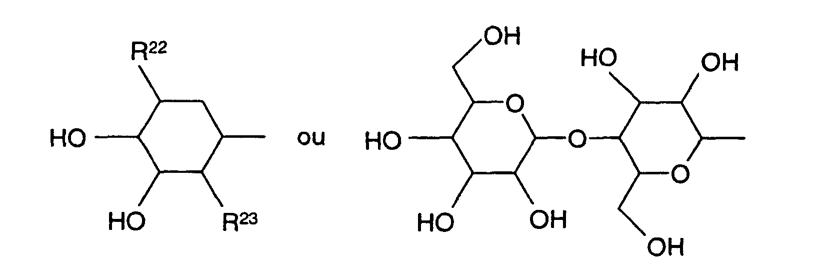 dans la forme D ou L,
où
R²¹ représente méthyle ou le groupe -CH₂OH et
R²² représente hydroxyle ou un reste de formule -NR²³R²⁴, où
R²³ et R²⁴ sont identiques ou différents et représentent l'hydrogène, alkyle linéaire ou ramifié ayant jusqu'à 6 atomes de carbone ou un groupe protecteur d'amino, et où les groupes hydroxyle des restes de sucre sont éventuellement protégés,
R¹⁸, R¹⁹ et R²⁰ sont identiques ou différents et représentent l'hydrogène ou alkyle linéaire ou ramifié ou cyclique ayant jusqu'à 8 atomes de carbone, qui est éventuellement substitué par phényle, halogène, azido, alcoxy, alcoxycarbonyle ou oxyacyle linéaire ou ramifié ayant dans chaque cas jusqu'à 6 atomes de carbone, hydroxyle, carboxyle, ou par un groupe de formule -NR²³R²⁴, où R²³ et R²⁴ ont la signification indiquée ci-dessus.

3. Composé de formule générale (I), (II), (III), (IV), (V), (VI) ou (VII) selon la revendication 1 ou 2, où
R¹ représente le chlore,
R² représente alkyle linéaire ou ramifié ayant jusqu'à 4 atomes de carbone, qui est éventuellement substitué par phényle, hydroxyle, alcoxy linéaire ou ramifié ayant jusqu'à 3 atomes de carbone,
R³ représente alkyle linéaire ou ramifié ayant jusqu'à 4 atomes de carbone, qui est éventuellement substitué par phényle, hydroxyle, alcoxy linéaire ou ramifié ayant jusqu'à 3 atomes de carbone, ou représente un groupe de formule -SO₂R⁸,
où
R⁸ représente méthyle, benzyle ou phényle, où ces derniers sont éventuellement substitués par méthyle ou éthyle,
ou bien
R³ représente un reste de formule 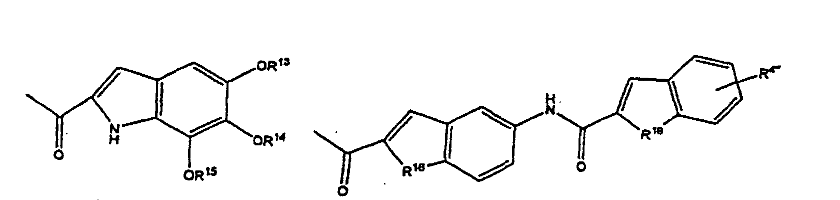 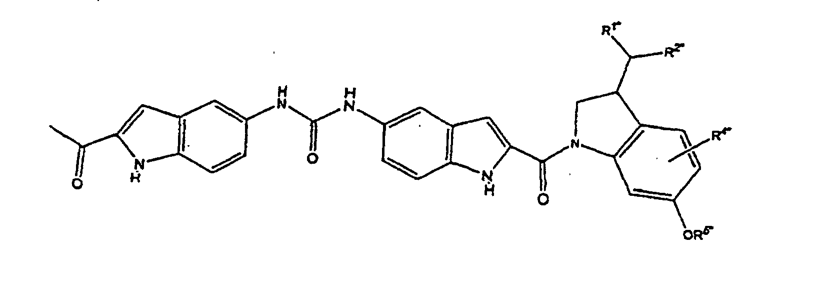 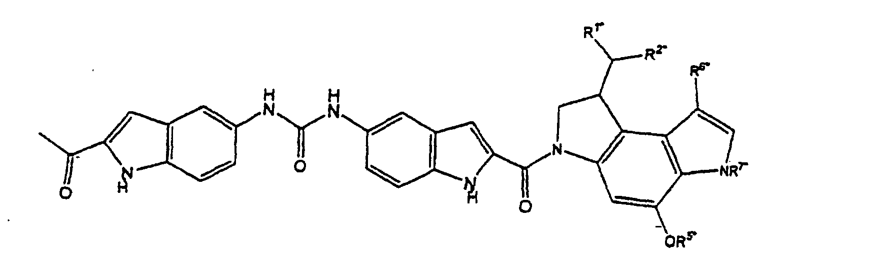 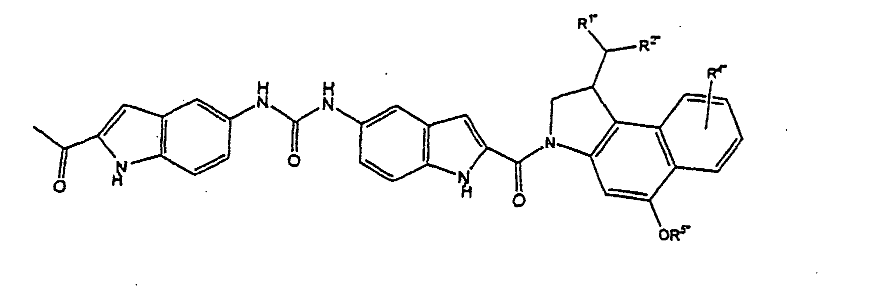 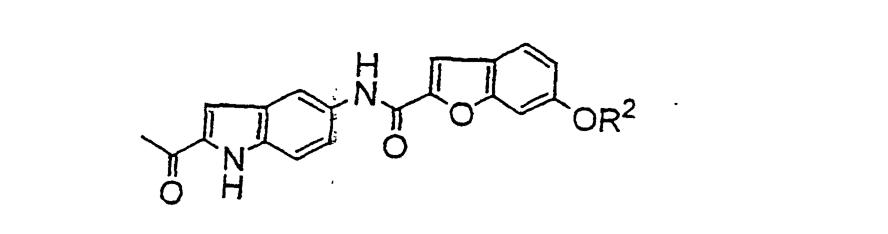 où
R^{1"}, R^{2"}, R^{4"}, R^{5"}, R^{6"} et R^{7"} ont les significations de R¹, R^{2'}, R⁴, R⁵, R⁶ et R⁷ indiquées ci-dessus et dans la suite et sont identiques ou différents de ceux-ci,
R¹³, R¹⁴ et R¹⁵ sont identiques ou différents et représentent l'hydrogène, méthyle, éthyle ou acétyle,
R¹⁶ représente l'azote, l'oxygène ou le soufre non substitué,
R⁴ représente l'hydrogène,
R⁵ représente l'α-D-mannose, le β-D-galactose, l'acide β-D-glucuronique et le β-D-glucose,
R⁶ et R⁷ sont identiques ou différents et représentent l'hydrogène ou un alkyle linéaire ou ramifié ayant jusqu'à 4 atomes de carbone.

4. Procédé de préparation d'un composé de formule générale (I), (II), (III), (IV), (V) ou (VI) selon l'une des revendications 1 à 3, **caractérisé en ce qu'**un composé de formule générale (IX), (X) ou (XI)
où 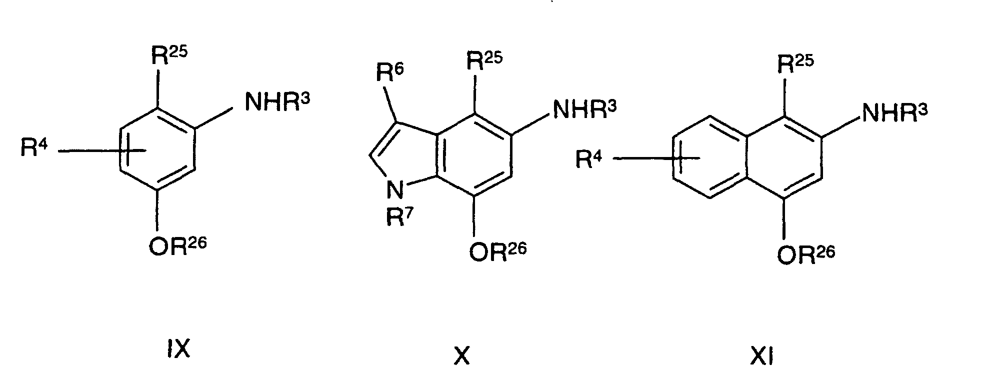 R³ a la signification indiquée ci-dessus, mais représente de préférence *tert*.-butoxycarbonyle,
R⁴, R⁶ et R⁷ ont la signification indiquée ci-dessus,
R²⁵ représente le brome ou l'iode,
R²⁶ représente l'un des groupes protecteurs d'hydroxyle indiqués ci-dessus, mais de préférence benzyle, benzyloxycarbonyle, 4-nitrobenzyle ou 4-méthoxybenzyle,
est converti après sa déprotonation sur l'azote au moyen d'une base appropriée avec un composé de formule générale (XII) ou (XIII),
où 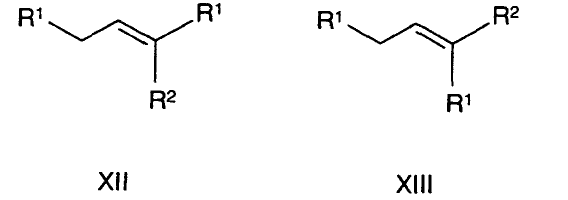 R¹ a la signification indiquée ci-dessus, mais représente de préférence le chlore,
R² a la signification indiquée ci-dessus,
en présence d'un solvant organique en un composé de formule générale (XIV), (XV), (XVI), (XVII), (XVIII) ou (XIX) 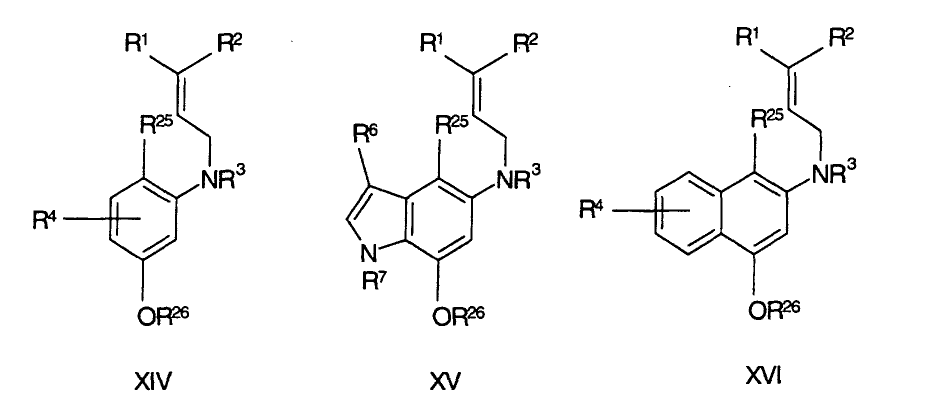 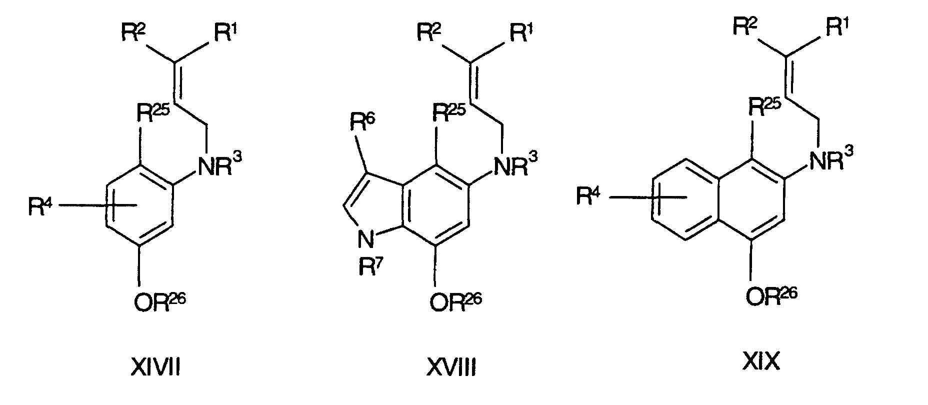 où
R¹ a la signification indiquée ci-dessus, mais représente de préférence le chlore,
R² a la signification indiquée ci-dessus,
R³ a la signification indiquée ci-dessus, mais représente de préférence *tert*.-butoxycarbonyle,
R⁴, R⁶, R⁷, R²⁵ et R²⁶ ont la signification indiquée ci-dessus,
et celui-ci est ensuite converti en un composé de formule générale (I), (II) ou (III) par une cyclisation radicalaire au moyen de l'hydrure de tributylétain et d'un initiateur radicalaire et finalement par clivage des groupes protecteurs sur l'oxygène phénolique par hydrogénolyse en présence de Pd/C et de formiate d'ammnonium,

5. Procédé selon la revendication, **caractérisé en ce que** R¹ représente le chlore dans la formule générale (I), (II) ou (III).

6. Procédé selon la revendication, **caractérisé en ce que** R² représente *tert*.-butoxycarbonyle dans la formule générale (I), (II) ou (III).

7. Procédé selon l'une des revendications 4 à 6 pour la préparation d'un composé de formule générale (IV), (V) ou (VI), **caractérisé en ce que** le composé de formule générale (I), (II) ou (III) est ensuite mis à réagir avec un mono-, di- ou oligosaccharide protégé, qui porte sur Ci un groupe trichloroacétimidate, un atome d'halogène ou un autre groupe partant, après quoi ll est éventuellement mis à réagir avec un acide carboxylique hétéroaromatique dans un solvant organique en présence d'un promoteur et pour différentes étapes de transformation en dérivé également en présence d'une base.

8. Procédé de préparation d'un composé de formule générale (VII) ou (VIII) selon la revendication 1, **caractérisé en ce qu'**un composé de formule générale (XX) 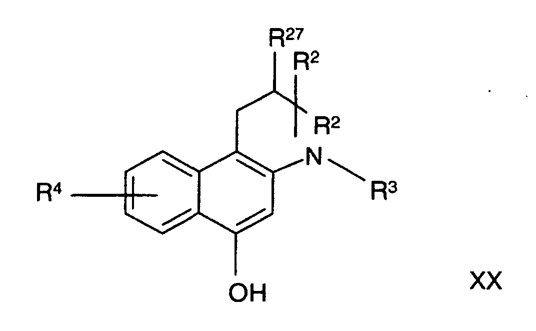 où
R², R³ et R⁴ ont la signification indiquée ci-dessus, et
R²⁷ a la signification de R¹ indiquée ci-dessus, mais représente de préférence le chlore ou
OSitBuPh₂ ou OSiOtBuPh₂ ou OSitBuMe₂ ou OCH₂Ph ou OCH₂-*p*-OMePh
dans le cas où R¹⁸ représente l'hydrogène dans la structure (VIII), est mis à réagir avec des énoléthers de formule générale (XXI) 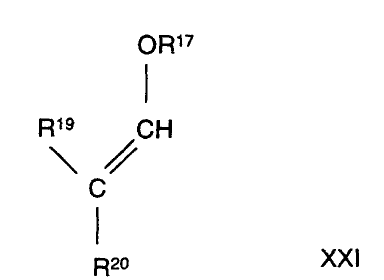 où
R¹⁷, R¹⁹ et R²⁰ ont la signification indiquée ci-dessus
et
dans le cas où R¹⁸ ≠ de l'hydrogène,
est mis à réagir avec des énoléthers de formule générale (XXIa) 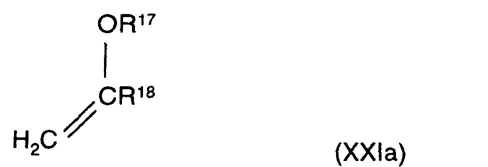 où
R¹⁷ et R¹⁸ ont la signification indiquée ci-dessus, ou avec un mono-, di- ou oligosaccharide protégé, qui porte en C₁ un groupe trichloroacétimidate ou un atome d'halogène ou un autre groupe partant, après quoi il est éventuellement mis à réagir avec un acide carboxylique hétéroaromatique dans un solvant organique en présence d'un catalyseur et pour différentes étapes de transformation en dérivé également en présence d'une base.

9. Agent pour la thérapie du cancer contenant un ou plusieurs composés selon l'une des revendications 1 à 3.

10. Utilisation d'un ou plusieurs composés selon l'une des revendications 1 à 3 pour la préparation d'agents pour la thérapie du cancer.
